# EUROPEAN PATENT SPECIFICATION

(11) **EP 1 849 866 B1**
(45) Date of publication and mention of the grant of the patent: **21.05.2008**
(21) Application number: 07008841.4
(22) Date of filing: 13.10.1999
(51) Int. Cl.: G01N 33/566, C07K 14/705

(54) **Human orphan G protein-coupled receptor RUP3**
Menschliche, an ein G-Protein gekoppelte Rezeptor RUP3 ohne bekannte Ligand
Récepteur humain orphelin RUP3 couplé à une protéine g

(30) Priority: 20.11.1998 US 109213 P; 16.02.1999 US 120416 P; 26.02.1999 US 121852 P; 12.03.1999 US 123946 P; 12.03.1999 US 123949 P; 28.05.1999 US 136436 P; 28.05.1999 US 136437 P; 28.05.1999 US 136439 P; 28.05.1999 US 136567 P; 28.05.1999 US 137127 P; 28.05.1999 US 137131 P; 29.06.1999 US 141448 P; 29.09.1999 US 156653 P; 29.09.1999 US 156633 P; 29.09.1999 US 156555 P; 29.09.1999 US 156634 P; 01.10.1999 US 157280 P; 01.10.1999 US 157294 P; 01.10.1999 US 157281 P; 01.10.1999 US 157293 P; 01.10.1999 US 157282 P; 12.10.1999 US 417044; 12.10.1999 US 416760
(43) Date of publication of application: 31.10.2007
(62) Divisional of application: 05003040.2
(73) Proprietor: Arena Pharmaceuticals, Inc., San Diego, CA 92121 (US)
(72) Inventor: Chen, Ruoping, San Diego CA 92130 (US); Leonard, James N., San Diego CA 92124 (US)
(74) Representative: Tollervey, Rebecca Marie

(56) References cited:
- EP-A- 1 092 727
- WO-A-00/22131
- WO-A-00/50562
- WO-A-98/31810
- STADEL J. M. ET AL.: "Orphan G protein-coupled receptors: A neglected opportunity for pioneer drug discovery." TRENDS IN PHARMACOLOGICAL SCIENCES, vol. 18, no. 11, November 1997 (1997-11), pages 430-437, XP004099345 ISSN: 0165-6147
- O'DOWD B. F. ET AL.: "DISCOVERY OF THREE NOVEL G-PROTEIN-COUPLED RECEPTOR GENES" GENOMICS, vol. 47, no. 2, 15 January 1998 (1998-01-15), pages 310-313, XP000863786 ISSN: 0888-7543
- MATSUOKA I. ET AL.: "Identification of novel members of G-protein coupled receptor subfamily" BIOCHEMICAL AND BIOPHYSICAL RESEARCH COMMUNICATIONS, vol. 194, no. 1, 15 July 1993 (1993-07-15), pages 504-511, XP002102959 ISSN: 0006-291X
- SCHEER A ET AL: "CONSTITUTIVELY ACTIVE G PROTEIN-COUPLED RECEPTORS: POTENTIAL MECHANISMS OF RECEPTOR ACTIVATION" JOURNAL OF RECEPTOR AND SIGNAL TRANSDUCTION RESEARCH, MARCEL DEKKER, NEW YORK, NY, US, vol. 17, no. 1/3, 1997, pages 57-73, XP000867531 ISSN: 1079-9893

## Description

### FIELD OF THE INVENTION

The invention disclosed in this patent document relates to transmembrane receptors, and more particularly to endogenous, orphan, human G protein-coupled receptors ("GPCRS"). The present invention relates only to Rup3; other GPCRs are discussed for reference only.

### BACKGROUND OF THE INVENTION

Although a number of receptor classes exist in humans, by far the most abundant and therapeutically relevant is represented by the G protein-coupled receptor (GPCR or GPCRs) class. It is estimated that there are some 100,000 genes within the human genome, and of these, approximately 2% or 2,000 genes, are estimated to code for GPCRs. Receptors, including GPCRs, for which the endogenous ligand has been identified are referred to as "known" receptors, while receptors for which the endogenous ligand has not been identified are referred to as "orphan" receptors. GPCRs represent an important area for the development of pharmaceutical products: from approximately 20 of the 100 known GPCRs, 60% of all prescription pharmaceuticals have been developed. This distinction is not merely semantic, particularly in the case of GPCRs. Thus, the orphan GPCRs are to the pharmaceutical industry what gold was to California in the late 19^{th} century - an opportunity to drive growth, expansion, enhancement and development.

GPCRs share a common structural motif. All these receptors have seven sequences of between 22 to 24 hydrophobic amino acids that form seven alpha helices, each of which spans the membrane (each span is identified by number, i.e., transmembrane-1 (TM-1), transmebrane-2 (TM-2), etc.). The transmembrane helices are joined by strands of amino acids between transmembrane-2 and transmembrane-3, transmembrane-4 and transmembrane-5, and transmembrane-6 and transmembrane-7 on the exterior, or "extracellular" side, of the cell membrane (these are referred to as "extracellular" regions 1, 2 and 3 (EC-1, EC-2 and EC-3), respectively). The transmembrane helices are also joined by strands of amino acids between transmembrane-1 and transmembrane-2, transmembrane-3 and transmembrane-4, and transmembrane-5 and transmembrane-6 on the interior, or "intracellular" side, of the cell membrane (these are referred to as "intracellular" regions 1, 2 and 3 (IC-1, IC-2 and IC-3), respectively). The "carboxy" ("C") terminus of the receptor lies in the intracellular space within the cell, and the "amino" ("N") terminus of the receptor lies in the extracellular space outside of the cell.

Generally, when an endogenous ligand binds with the receptor (often referred to as "activation" of the receptor), there is a change in the conformation of the intracellular region that allows for coupling between the intracellular region and an intracellular "G-protein." It has been reported that GPCRs are "promiscuous with respect to G proteins, i.e., that a GPCR can interact with more than one G protein. See, Kenakin, T., 43 Life Sciences 1095 (1988). Although other G proteins exist, currently, Gq, Gs, Gi, and Go are G proteins that have been identified. Endogenous ligand-activated GPCR coupling with the G-protein begins a signaling cascade process (referred to as "signal transduction"). Under normal conditions, signal transduction ultimately results in cellular activation or cellular inhibition. It is thought that the IC-3 loop as well as the carboxy terminus of the receptor interact with the G protein.

Under physiological conditions, GPCRs exist in the cell membrane in equilibrium between two different conformations an "inactive" state and an "active" state. A receptor in an inactive state is unable to link to the intracellular signaling transduction pathway to produce a biological response. Changing the receptor conformation to the active state allows linkage to the transduction pathway (via the G-protein) and produces a biological response. A receptor may be stabilized in an active state by an endogenous ligand or a compound such as a drug.

### SUMMARY OF THE INVENTION

The present invention provides a method of screening candidate compounds to identify a pharmaceutical agent for a disease or disorder state related to the pancreas, the method comprising:
providing a membrane of a cell expressing a G protein-coupled receptor which is a ligand-independent active version of a receptor having SEQ ID NO: 8, wherein the G protein-coupled receptor couples to a G-protein; and
screening candidate compounds against said G protein-coupled receptor.

### BRIEF DESCRIPTION OF THE DRAWINGS

**Figures 1A** and **1B** provide reference "grids" for certain dot-blots provided herein (*see also,* Figure 2A and 2B, respectively).
**Figures 2A** and **2B** provide reproductions of the results of certain dot-blot analyses resulting from hCHN3 and hCHN8, respectively (*see also,* Figures 1A and 1B, respectively).
**Figure 3** provides a reproduction of the results of RT-PCR analysis of hRUP3.
**Figure 4** provides a reproduction of the results of RT-PCR analysis of hRUP4.
**Figure 5** provides a reproduction of the results of RT-PCR analysis of hRUP6.

### DETAILED DESCRIPTION

The scientific literature that has evolved around receptors has adopted a number of terms to refer to ligands having various effects on receptors. For clarity and consistency, the following definitions will be used throughout this patent document. To the extent that these definitions conflict with other definitions for these terms, the following definitions shall control:
**AMINO ACID ABBREVIATIONS** used herein are set out in Table 1:

| **TABLE 1** | | |
|---|---|---|
| ALANINE | ALA | A |
| ARGININE | ARG | R |
| ASPARAGINE | ASN | N |
| ASPARTIC ACID | ASP | D |
| CYSTEINE | CYS | C |
| GLUTAMIC ACID | GLU | E |
| GLUTAMINE | GLN | Q |
| GLYCINE | GLY | G |
| HISTIDINE | HIS | H |
| ISOLEUCINE | ILE | I |
| LEUCINE | LEU | L |
| LYSINE | LYS | K |
| METHIONINE | MET | M |
| PHENYLALANINE | PHE | F |
| PROLINE | PRO | P |
| SERINE | SER | S |
| THREONINE | THR | T |
| TRYPTOPHAN | TRP | W |
| TYROSINE | TYR | Y |
| VALINE | VAL | V |

**COMPOSITION** means a material comprising at least one component.
**ENDOGENOUS** shall mean a material that a mammal naturally produces. ENDOGENOUS in reference to, for example and not limitation, the term "receptor," shall mean that which is naturally produced by a mammal (for example, and not limitation, a human) or a virus. By contrast, the term **NON-ENDOGENOUS** in this context shall mean that which is not naturally produced by a mammal (for example, and not limitation, a human) or a virus.
**HOST CELL** shall mean a cell capable of having a Plasmid and/or Vector incorporated therein. In the case of a prokaryotic Host Cell, a Plasmid is typically replicated as a autonomous molecule as the Host Cell replicates (generally, the Plasmid is thereafter isolated for introduction into a eukaryotic Host Cell); in the case of a eukaryotic Host Cell, a Plasmid is integrated into the cellular DNA of the Host Cell such that when the eukaryotic Host Cell replicates, the Plasmid replicates. The Host Cell may be eukaryotic, more preferably, mammalian, and most preferably selected from the group consisting of 293, 293T and COS-7 cells.
**LIGAND** shall mean an endogenous, naturally occurring molecule specific for an endogenous, naturally occurring receptor.
**NON-ORPHAN RECEPTOR** shall mean an endogenous naturally occurring molecule specific for an endogenous naturally occurring ligand wherein the binding of a ligand to a receptor activates an intracellular signaling pathway.
**ORPHAN RECEPTOR** shall mean an endogenous receptor for which the endogenous ligand specific for that receptor has not been identified or is not known.
**PLASMID** shall mean the combination of a Vector and cDNA, Generally, a Plasmid is introduced into a Host Cell for the purposes of replication and/or expression of the cDNA as a protein.
**VECTOR** sin reference to cDNA shall mean a circular DNA capable of incorporating at least one cDNA and capable of incorporation into a Host Cell.

The order of the following sections is set forth for presentational efficiency and is not intended, nor should be construed, as a limitation on the disclosure or the claims to follow.

### A. Identification of Human GPCRs

The efforts of the Human Genome project have led to the identification of a plethora of information regarding nucleic acid sequences located within the human genome; it has been the case in this endeavor that genetic sequence information has been made available without an understanding or recognition as to whether or not any particular genomic sequence does or may contain open-reading frame information that translate human proteins. Several methods of identifying nucleic acid sequences within the human genome are within the purview of those having ordinary skill in the art. For example, and not limitation, a variety of GPCRs, disclosed herein, were discovered by reviewing the GenBank^{™} database, while other GPCRs were discovered by utilizing a nucleic acid sequence of a GPCR, previously sequenced, to conduct a BLAST^{™} search of the EST database. **Table A,** below, lists the disclosed endogenous orphan GPCRs along with a GPCR's respective homologous GPCR:

**TABLE A**

| **Disclosed** **Human** **Orphan** **GPCRs** | **Accession** **Number** **Identified** | **Open Rearing** **Frame** **(Base Pairs)** | **Per Cent** **Hontology** **To** **Designated** **GPCR** | **Reference** **To** **Homologous** **GPCR** **(Accession** **No.)** |
|---|---|---|---|---|
| **hAKE-3** | AL033379 | 1,260 bp | 52.3% LPA-R | U92642 |
| **hARE-4** | AC006087 | 1,119 bp | 36% P2Y5 | AF000546 |
| **hARE-5** | AC006255 | 1,104 bp | 32% | D43633 |
| | | | *Oryzias* | |
| | | | *latipes* | |
| **hGPR27** | AA775870 | 1,128 bp | | |
| **hARE-1** | AI090920 | 1,002 bp | 43% | D13626 |
| | | | KIAA0001 | |
| **hARE-2** | AA359504 | 1,122 bp | 53% GPR27 | |
| **hPPR1** | H67224 | 1,053 bp | 39% EBI1 | L31581 |
| **hG2A** | AA754702 | 1,116 bp | 31% GPR4 | L36148 |
| **hRUP3** | AL035423 | 1,008 bp | 30% | 2133653 |
| | | | *Drosophilia* | |
| | | | *melanogaster* | |
| **hRUP4** | AI307658 | 1,296 bp | 32% pNPGPR | NP_004876 |
| | | | 28% and 29 | AAC41276 |
| | | | % *Zebra* | and |
| | | | *fish* Ya | AAB94616 |
| | | | and Yb, | |
| | | | respectively | |
| **hRUP5** | AC005849 | 1,413 bp | 25% DEZ | Q99788 |
| | | | 23% FMLPR | P21462 |
| **hRUP36** | AC005871 | 1,245 bp | 48% GPR66 | NP_006047 |
| **hRUP7** | AC007922 | 1,173 bp | 43% H3R | AF140538 |
| **hCHN3** | EST 36581 | 1,113 bp | 53% GPR27 | |
| **hCHN4** | AA804531 | 1,080 bp | 32% | 4503637 |
| | | | thrombin | |
| **hCHN6** | EST 2134670 | 1,503 bp | 36% edg-1 | NP_001391 |
| **hCHN8** | EST 764455 | 1,029 bp | 47% | D13626 |
| | | | KIAA0001 | |
| **hCHN9** | EST 1541536 | 1,077 bp | 41% LTB4R | NM_000752 |
| **hCHN10** | EST 1365839 | 1,055 bp | 35% P2Y | NM_002563 |

Receptor homology is useful in terms of gaining an appreciation of a role of the disclosed receptors within the human body. Additionally, such homology can provide insight as to possible endogenous ligand(s) that may be natural activators for the disclosed orphan GPCRs.

### B. Receptor Screening

Techniques have become more readily available over the past few years for endogenous-ligand identification (this, primarily, for the purpose of providing a means of conducting receptor-binding assays that require a receptor's endogenous ligand) because the traditional study of receptors has always proceeded from the a priori assumption (historically based) that the endogenous ligand must first be identified before discovery could proceed to find antagonists and other molecules that could affect the receptor. Even in cases where an antagonist might have been known first, the search immediately extended to looking for the endogenous ligand. This mode of thinking has persisted in receptor research even after the discovery of constitutively activated receptors. What has not been heretofore recognized is that it is the active state of the receptor that is most useful for discovering agonists, partial agonists, and inverse agonists of the receptor. For those diseases which result from an overly active receptor or an under-active receptor, what is desired in a therapeutic drug is a compound which acts to diminish the active state of a receptor or enhance the activity of the receptor, respectively, not necessarily a drug which is an antagonist to the endogenous ligand. This is because a compound that reduces or enhances the activity of the active receptor state need not bind at the same site as the endogenous ligand. Thus, as taught by a method of this invention, any search for therapeutic compounds should start by screening compounds against the ligand-independent active state.

As is known in the art, GPCRs can be "active" in their endogenous state even without the binding of the receptor's endogenous ligand thereto. Such naturally-active receptors can be screened for the direct identification (*i*.*e*., without the need for the receptor's endogenous ligand) of, in particular, inverse agonists. Alternatively, the receptor can be "activated" via, *e*.*g*., mutation of the receptor to establish a non-endogenous version of the receptor that is active in the absence of the receptor's endogenous ligand.

Screening candidate compounds against an endogenous or non-endogenous, constitutively activated version of the human orphan GPCRs disclosed herein can provide for the direct identification of candidate compounds which act at this cell surface receptor, without requiring use of the receptor's endogenous ligand. By determining areas within the body where the endogenous version of human GPCRs disclosed herein is expressed and/or over-expressed, it is possible to determine related disease/disorder states which are associated with the expression and/or over-expression of the receptor; such an approach is disclosed in this patent document.

With respect to creation of a mutation that may evidence constitutive activation of human orphan GPCRs disclosed herein is based upon the distance from the proline residue at which is presumed to be located within TM6 of the GPCR typically nears the TM6/IC3 interface (such proline residue appears to be quite conserved). By mutating the amino acid residue located 16 amino acid residues from this residue (presumably located in the IC3 region of the receptor) to, most preferably, a lysine residue, such activation may be obtained. Other amino acid residues may be useful in the mutation at this position to achieve this objective.

### C. Disease/Disorder Identification and/or Selection

Preferably, the DNA sequence of the human orphan GPCR can be used to make a probe for (a) dot-blot analysis against tissue-mRNA, and/or (b) RT-PCR identification of the expression of the receptor in tissue samples. The presence of a receptor in a tissue source, or a diseased tissue, or the presence of the receptor at elevated concentrations in diseased tissue compared to a normal tissue, can be preferably utilized to identify a correlation with a treatment regimen, including but not limited to, a disease associated with that disease. Receptors can equally well be localized to regions of organs by this technique. Based on the known functions of the specific tissues to which the receptor is localized, the putative functional role of the receptor can be deduced.

### D. Screening of Candidate Compounds

### 1. Generic GPCR screening assay techniques

When a G protein receptor becomes constitutively active (i.e., active in the absence of endogenous ligand binding thereto), it binds to a G protein (e.g., Gq, Gs, Gi, Go) and stimulates the binding of GTP to the G protein. The G protein then acts as a GTPase and slowly hydrolyzes the GTP to GDP, whereby the receptor, under normal conditions, becomes deactivated. However, constitutively activated receptors continue to exchange GDP to GTP. A non-hydrolyzable analog of GTP, [³⁵S]GTPγS, can be used to monitor enhanced binding to membranes which express constitutively activated receptors. It is reported that [³⁵S]GTPγS can be used to monitor G protein coupling to membranes in the absence and presence of ligand. An example of this monitoring, among other examples well-known and available to those in the art, was reported by Traynor and Nahorski in 1995. The preferred use of this assay system is for initial screening of candidate compounds because the system is generically applicable to all G protein-coupled receptors regardless of the particular G protein that interacts with the intracellular domain of the receptor.

### 2. Specific GPCR screening assay techniques

Once candidate compounds are identified using the "generic" G protein-coupled receptor assay (i.e., an assay to select compounds that are agonists, partial agonists, or inverse agonists), further screening to confirm that the compounds have interacted at the receptor site is preferred. For example, a compound identified by the "generic" assay may not bind to the receptor, but may instead merely "uncouple" the G protein from the intracellular domain.

### a. Gs and Gi.

Gs stimulates the enzyme adenylyl cyclase. Gi (and Go), on the other hand, inhibit this enzyme. Adenylyl cyclase catalyzes the conversion of ATP to cAMP; thus, constitutively activated GPCRs that couple the Gs protein are associated with increased cellular levels of cAMP. On the other hand, constitutively activated GPCRs that couple the Gi (or Go) protein are associated with decreased cellular levels of cAMP. *See, generally,* "Indirect Mechanisms of Synaptic Transmission," Chpt. 8, From Neuron To Brain (3rd Ed.) Nichols, J.G. et al eds. Sinauer Associates, Inc. (1992). Thus, assays that detect cAMP can be utilized to determine if a candidate compound is, *e*.*g*., an inverse agonist to the receptor (*i*.*e*., such a compound would decrease the levels of cAMP). A variety of approaches known in the art for measuring cAMP can be utilized; a most preferred approach relies upon the use of anti-cAMP antibodies in an ELISA-based format. Another type of assay that can be utilized is a whole cell second messenger reporter system assay. Promoters on genes drive the expression of the proteins that a particular gene encodes. Cyclic AMP drives gene expression by promoting the binding of a cAMP-responsive DNA binding protein or transcription factor (CREB) which then binds to the promoter at specific sites called cAMP response elements and drives the expression of the gene. Reporter systems can be constructed which have a promoter containing multiple cAMP response elements before the reporter gene, *e*.*g*., β-galactosidase or luciferase. Thus, a constitutively activated Gs-linked receptor causes the accumulation of cAMP that then activates the gene and expression of the reporter protein. The reporter protein such as β-galactosidase or luciferase can then be detected using standard biochemical assays (Chen et al. 1995).

### b. Go and Gq.

Gq and Go are associated with activation of the enzyme phospholipase C, which in turn hydrolyzes the phospholipid PIP₂, releasing two intracellular messengers: diacycloglycerol (DAG) and inistol 1,4,5-triphoisphate (IP₃). Increased accumulation of IP₃ is associated with activation of Gq- and Go-associated receptors. *See, generally*, "Indirect Mechanisms of Synaptic Transmission," Chpt. 8, From Neuron To Brain (3rd Ed.) Nichols, J.G. et al eds. Sinauer Associates, Inc. (1992). Assays that detect IP₃ accumulation can be utilized to determine if a candidate compound is, *e*.*g*., an inverse agonist to a Gq- or Go-associated receptor (*i*.*e*., such a compound would decrease the levels of IP₃). Gq-associated receptors can also been examined using an AP1 reporter assay in that Gq-dependent phospholipase C causes activation of genes containing AP1 elements; thus, activated Gq-associated receptors will evidence an increase in the expression of such genes, whereby inverse agonists thereto will evidence a decrease in such expression, and agonists will evidence an increase in such expression. Commercially available assays for such detection are available.

### 3. GPCR Fusion Protein

The use of an endogenous, constitutively activated orphan GPCR, or a non-endogenous, constitutively activated orphan GPCR, for screening of candidate compounds for the direct identification of inverse agonists, agonists and partial agonists provides a unique challenge in that, by definition, the receptor is active even in the absence of an endogenous ligand bound thereto. Thus, it is often useful that an approach be utilized that can enhance the signal obtained by the activated receptor. A preferred approach is the use of a GPCR Fusion Protein.

Generally, once it is determined that a GPCR is or has been constitutively activated, using the assay techniques set forth above (as well as others), it is possible to determine the predominant G protein that couples with the endogenous GPCR. Coupling of the G protein to the GPCR provides a signaling pathway that can be assessed. Because it is most preferred that screening take place by use of a mammalian expression system, such a system will be expected to have endogenous G protein therein. Thus, by definition, in such a system, the constitutively activated orphan GPCR will continuously signal. In this regard, it is preferred that this signal be enhanced such that in the presence of, e.g., an inverse agonist to the receptor, it is more likely that it will be able to more readily differentiate, particularly in the context of screening, between the receptor when it is contacted with the inverse agonist.

The GPCR Fusion Protein is intended to enhance the efficacy of G protein coupling with the GPCR. The GPCR Fusion Protein is preferred for screening with a non-endogenous, constitutively activated GPCR because such an approach increases the signal that is most preferably utilized in such screening techniques, although the GPCR Fusion Protein can also be (and preferably is) used with an endogenous, constitutively activated GPCR. This is important in facilitating a significant "signal to noise" ratio;
such a significant ratio is import preferred for the screening of candidate compounds as disclosed herein.

The construction of a construct useful for expression of a GPCR Fusion Protein is within the purview of those having ordinary skill in the art. Commercially available expression vectors and systems offer a variety of approaches that can fit the particular needs of an investigator. The criteria of importance for such a GPCR Fusion Protein construct is that the GPCR sequence and the G protein sequence both be in-frame (preferably, the sequence for the GPCR is upstream of the G protein sequence) and that the "stop" codon of the GPCR must be deleted or replaced such that upon expression of the GPCR, the G protein can also be expressed. The GPCR can be linked directly to the G protein, or there can be spacer residues between the two (preferably, no more than about 12, although this number can be readily ascertained by one of ordinary skill in the art). We have a preference (based upon convenience) of use of a spacer in that some restriction sites that are not used will, effectively, upon expression, become a spacer. Most preferably, the G protein that couples to the GPCR will have been identified prior to the creation of the GPCR Fusion Protein construct. Because there are only a few G proteins that have been identified, it is preferred that a construct comprising the sequence of the G protein (*i*.*e*., a universal G protein construct) be available for insertion of an endogenous GPCR sequence therein; this provides for efficiency in the context of large-scale screening of a variety of different endogenous GPCRs having different sequences.

### E. Other Utility

A preferred use of the human orphan GPCRs disclosed herein may be for the direct identification of candidate compounds as inverse agonists, agonists or partial agonists (preferably for use as pharmaceutical agents). These versions of human GPCRs can also be utilized in research settings. For example, *in vitro* and *in vivo* systems incorporating GPCRs can be utilized to further elucidate and understand the roles these receptors play in the human condition, both normal and diseased, as well as understanding the role of constitutive activation as it applies to understanding the signaling cascade. The value in human orphan GPCRs is that its utility as a research tool is enhanced in that by determining the location(s) of such receptors within the body, the GPCRs can be used to understand the role of these receptors in the human body before the endogenous ligand therefor is identified. Other uses of the disclosed receptors will become apparent to those in the art based upon, *inter alia*, a review of this patent document.

### EXAMPLES

The following examples are presented for purposes of elucidation, and not limitation, of the present invention and for the provision of reference information. While specific nucleic acid and amino acid sequences are disclosed herein, those of ordinary skill in the art are credited with the ability to make minor modifications to these sequences while achieving the same or substantially similar results reported below. Unless otherwise indicated below, all nucleic acid sequences for the disclosed endogenous orphan human GPCRs have been sequenced and verified. For purposes of equivalent receptors, those of ordinary skill in the art will readily appreciate that conservative substitutions can be made to the disclosed sequences to obtain a functionally equivalent receptor.

### Example 1

### ENDOGENOUS HUMAN GPCRS

### 1. Identification of Human GPCRs

Several of the disclosed endogenous human GPCRs were identified based upon a review of the GenBank database information. While searching the database, the following cDNA clones were identified as evidenced below.

| **Disclosed** **Human** **Orphan** **GPCRs** | **Accession** **Number** | **Complete DNA** **Sequence** **(Base Pairs)** | **Open Reading** **Frame** **(Base Pairs)** | **Nucleic** **Acid** **SEQ.ID.** **NO.** | **Amino** **Acid** **SEQ.ID.** **NO.** |
|---|---|---|---|---|---|
| **hARE-3** | AL033379 | 111, 389 bp | 1,260 bp | 1 | 2 |
| **hARE-4** | AC006087 | 226,925 bp | 1,119 bp | 3 | 4 |
| **hARE-5** | AC006255 | 127,605 bp | 1,104 bp | 5 | 6 |
| **hRUP3** | AL035423 | 140,094 bp | 1,008 bp | 7 | 8 |
| **hRUP5** | AC005849 | 169,144 bp | 1,413 bp | 9 | 10 |
| **hRUP6** | AC005871 | 218,807 bp | 1,245 bp | 11 | 12 |
| **hRUP7** | AC007922 | 158,858 bp | 1,173 bp | 13 | 14 |

Other disclosed endogenous human GPCRs were identified by conducting a BLAST search of EST database (dbest) using the following EST clones as query sequences. The following EST clones identified were then used as a probe to screen a human genomic library.

| **Disclosed** **Human** **Orphan GPCRs** | **Query** **(Sequence)** | **EST Clone/** **Accession** **No.** **Identified** | **Open** **Reading** **Frame** **(Base** **Pairs)** | **Nucleic** **Acid** **SEQ.ID.NO.** | **Amino Acid** **SEQ.ID.NO.** |
|---|---|---|---|---|---|
| **hGPCR27** | Mouse GPCR27 | AA775870 | 1,128 bp | 15 | 16 |
| **hARR-1** | TDAG | 1689643 | 1,002 bp | 17 | 18 |
| | | AI090920 | | | |
| **hARE-2** | GPCR27 | 68530 | 1,122 bp | 19 | 20 |
| | | AA359504 | | | |
| **hPPR1** | Bovine | 238667 | 1.053 bp | 21 | 22 |
| | PPR1 | H67224 | | | |
| **hG2A** | Mouse | *See* | 1,116 bp | 23 | 24 |
| | 1179426 | *Example* | | | |
| | | *2 (a)*, | | | |
| | | *below* | | | |
| **hCHN3** | N.A. | EST 36581 | 1,113 bp | 25 | 26 |
| | | (full | | | |
| | | length) | | | |
| **hCHN4** | TDAG | 1184934 | 1,080 bp | 27 | 28 |
| | | AA804531 | | | |
| **hCHN6** | N.A. | EST | 1, 503 bp | 29 | 30 |
| | | 2134670 | | | |
| | | (full | | | |
| | | length) | | | |
| **hCHN8** | KIAA0001 | EST 764455 | 1,029 bp | 31 | 32 |
| **hCBN 9** | 1365839 | EST | 1,077 bp | 33 | 34 |
| | | 1541536 | | | |
| **hCHN10** | Mouse EST | Human | 1,005 bp | 35 | 36 |
| | 1365839 | 1365839 | | | |
| **hRUP4** | N.A. | AI307658 | 1,296 bp | 37 | 38 |

| | | | | | |
|---|---|---|---|---|---|
| *N*.*A*. = "*not applicable*". | | | | | |

### 2. Full Length Cloning

### a. hG2A (Seq. Id. Nos. 23 & 24)

Mouse EST clone 1179426 was used to obtain a human genomic clone containing all but three amino acid hG2A coding sequences. The 5' end of this coding sequence was obtained by using 5'RACE^{™}, and the template for PCR was Clontech's Human Spleen Marathon-ready^{™} cDNA. The disclosed human G2A was amplified by PCR using the G2A cDNA specific primers for the first and second round PCR as shown in SEQ.ID.NO.: 39 and SEQ.ID.NO.:40 as follows: 5'-CTGTGTACAGCAGTTCGCAGAGTG-3' (SEQ.ID.NO.: 39; 1^{st} round PCR) 5'-GAGTGCCAGGCAGAGCAGGTAGAC-3' (SEQ.ID.NO.: 40; second round PCR). PCR was performed using Advantage^{™} GC Polymerase Kit (Clontech; manufacturing instructions will be followed), at 94°C for 30 sec followed by 5 cycles of 94°C for 5 sec and 72°C for 4 min; and 30 cycles of 94° for 5 sec and 70° for 4 min. An approximate 1.3 Kb PCR fragment was purified from agarose gel, digested with Hind III and Xba I and cloned into the expression vector pRC/CMV2 (Invitrogen). The cloned-insert was sequenced using the T7 Sequenase^{™} kit (USB Amersham; manufacturer instructions will be followed) and the sequence was compared with the presented sequence. Expression of the human G2A will be detected by probing an RNA dot blot (Clontech; manufacturer instructions will be followed) with the P³²-labeled fragment.

### b.hCHN9 (Seq. Id. Nos. 33 & 34)

Sequencing of the EST clone 1541536 indicated that hCHN9 is a partial cDNA clone having only an initiation codon; i.e., the termination codon was missing. When hCHN9 was used to "blast" against the data base (nr), the 3' sequence of hCHN9 was 100% homologous to the 5' untranslated region of the leukotriene B4 receptor cDNA, which contained a termination codon in the frame with hCHN9 coding sequence. To determine whether the 5' untranslated region of LTB4R cDNA was the 3' sequence of hCHN9, PCR was performed using primers based upon the 5' sequence flanking the initiation codon found in hCHN9 and the 3' sequence around the termination codon found in the LTB4R 5' untranslated region. The 5' primer sequence utilized was as follows:
5'-CCCGAATTCCTGCTTGCTCCCAGCTTGGCCC-3' (SEQ.ID.NO.: 41; sense) and
5'-TGTGGATCCTGCTGTCAAAGGTCCCATTCCGG-3' (SEQ.ID.NO.: 42; antisense).
PCR was performed using thymus cDNA as a template and rTth polymerase (Perkin Elmer) with the buffer system provided by the manufacturer, 0.25 uM of each primer, and 0.2 mM of each 4 nucleotides. The cycle condition was 30 cycles of 94°C for 1 min, 65°C for 1min and 72 °C for 1 min and 10 sec. A 1.1kb fragment consistent with the predicted size was obtained from PCR. This PCR fragment was subcloned into pCMV (see below) and sequenced (*see,* SEQ.ID.NO.: 33).

### c. HRUP 4 (Seq. Id. Nos. 37 & 38)

The full length hRUP4 was cloned by RT-PCR with human brain cDNA (Clontech) as templates:
5'-TCACAATGCTAGGTGTGGTC-3' (SEQ.ID.NO.: 43; sense) and
5'-TGCATAGACAATGGGATTACAG-3' (SEQ.ID.NO.: 44; antisense).
PCR was performed using TaqPlus^{™} Precision^{™} polymerase (Stratagene; manufacturing instructions will be followed) by the following cycles: 94°C for 2 min; 94°C 30 sec; 55°C for 30 sec, 72°C for 45 sec, and 72°C for 10 min. Cycles 2 through 4 were repeated 30 times.

The PCR products were separated on a 1% agarose gel and a 500 bp PCR fragment was isolated and cloned into the pCRII-TOPO vector (Invitrogen) and sequenced using the T7 DNA Sequenase^{™} kit (Amsham) and the SP6/T7 primers (Stratagene). Sequence analysis revealed that the PCR fragment was indeed an alternatively spliced form of AI307658 having a continuous open reading frame with similarity to other GPCRs. The completed sequence of this PCR fragment was as follows:

Based on the above sequence, two sense oligonucleotide primer sets:
5'-CTGCTTAGAAGAGTGGACCAG-3' (SEQ.ID.NO.: 46; oligo 1),
5'-CTGTGCACCAGAAGATCTACAC-3' (SEQ.IDNO.: 47; oligo 2)
and two antisense oligonucleotide primer sets:
5'-CAAGGATGAAGGTGGTGTAGA-3' (SEQ.ID.NO.: 48; oligo 3)
5'-GTGTAGATCTTCTGGTGCACAGG-3' (SEQ.ID.NO.: 49; oligo 4)
were used for 3'- and 5'-race PCR with a human brain Marathon-Ready^{™} cDNA (Clontech, Cat# 7400-1) as template, according to manufacture's instructions. DNA fragments generated by the RACE PCR were cloned into the pCRII-TOPO^{™} vector (Invitrogen) and sequenced using the SP6/T7 primers (Stratagene) and some internal primers. The 3' RACE product contained a poly(A) tail and a completed open reading frame ending at a TAA stop codon. The 5' RACE product contained an incomplete 5' end; *i*.*e*., the ATG initiation codon was not present.

Based on the new 5' sequence, oligo 3 and the following primer:
5'-GCAATGCAGGTCATAGTGAGC -3' (SEQ.ID.NO.: 50; oligo 5)
were used for the second round of 5' RACE PCR and the PCR products were analyzed as above. A third round of 5' RACE PCR was carried out utilizing antisense primers:
5'-TGGAGCATGGTGACGGGAATGCAGAAG-3' (SEQ.ID.NO.: 51; oligo 6) and
5'-GTGATGAGCAGGTCACTGAGCGCCAAG-3' (SEQ.ID.NO.: 52; oligo7).

The sequence of the 5' RACE PCR products revealed the presence of the initiation codon ATG, and further round of 5' RACE PCR did not generate any more 5' sequence. The completed 5' sequence was confirmed by RT-PCR using sense primer 5'-GCAATGCAGGCGCTTAACATTAC-3' (SEQ.ID.NO.: 53; oligo 8) and oligo 4 as primers and sequence analysis of the 650 bp PCR product generated from human brain and heart cDNA templates (Clontech, Cat# 7404-1). The completed 3' sequence was confirmed by RT-PCR using oligo 2 and the following antisense primer:
5'-TTGGGTTACAATCTGAAGGGCA-3' (SEQ.ID.NO.: 54; oligo 9)
and sequence analysis of the 670 bp PCR product generated from human brain and heart cDNA templates. (Clontech, Cat# 7404-1).

### d. hRUP5 (Seq. Id. Nos. 9 & 10)

the full length hRUP5 was cloned by RT-PCR using a sense primer upstream from ATG, the initiation codon (SEQ.ID.NO.: 55), and an antisense primer containing TCA as the stop codon (SEQ.ID.NO.: 56), which had the following sequences:
5'-ACTCCGTGTCCAGCAGGACTCTG-3' (SEQ.ID.NO.:55)
5'-TGCGTGTTCCTGGACCCTCACGTG-3' (SEQ.ID.NO.: 56)
and human peripheral leukocyte cDNA (Clontech) as a template. Advantage cDNA polymerase (Clontech) was used for the amplification in a 50ul reaction by the following cycle with step 2 through step 4 repeated 30 times: 94°C for 30 sec; 94° for 15 sec; 69° for 40 sec; 72°C for 3 min; and 72°C fro 6 min. A 1.4kb PCR fragment was isolated and cloned with the pCRII-TOPO^{™} vector (Invitrogen) and completely sequenced using the T7 DNA Sequenase^{™} kit (Amsham). See, SEQ.ID.NO.: 9.

### e. hRUP6 (Seq. Id. Nos. 11 & 12)

The full length hRUP6 was cloned by RT-PCR using primers:
5'-CAGGCCTTGGATTTTAATGTCAGGGATGG-3' (SEQ.ID.NO.: 57) and
5'-GGAGAGTCAGCTCTGAAAGAATTCAGG-3' (SEQ.ID.NO.: 58);
and human thymus Marathon-Ready^{™} cDNA (Clontech) as a template. Advantage cDNA polymerase (Clontech, according to manufacturer's instructions) was used for the amplification in a 50ul reaction by the following cycle: 94°C for 30sec; 94°C for 5 sec; 66°C for 40sec; 72°C for 2.5 sec and 72°C for 7 min. Cycles 2 through 4 were repeated 30 times. A 1.3 Kb PCR fragment was isolated and cloned into the pCRII-TOPO^{™} vector (Invitrogen) and completely sequenced (see, SEQ.ID.NO.: 11) using the ABI Big Dye Terminator^{™} kit (P.E. Biosystem).

### f.hRUP7 (Seq. Id. Nos. 13 & 14)

The full length RUP7 was cloned by RT-PCR using primers:
5'-TGATGTGATGCCAGATACTAATAGCAC-3' (SEQ.ID.NO.: 59; sense) and
5'-CCTGATTCATTTAGGTGAGATTGAGAC-3' (SEQ.ID.NO.: 60; antisense)
and human peripheral leukocyte cDNA (Clontech) as a template. Advantage^{™} cDNA polymerase (Clontech) was used for the amplification in a 50 ul reaction by the following cycle with step 2 to step 4 repeated 30 times: 94°C for 2 minutes; 94°C for 15 seconds; 60°C for 20 seconds; 72°C for 2 minutes; 72°C for 10 minutes. A 1.25 Kb PCR fragment was isolated and cloned into the pCRII-TOPO^{™} vector (Invitrogen) and completely sequenced using the ABI Big Dye Terminator^{™} kit (P.E. Biosystem). *See,* SEQ.ID.NO.: 13.

### g.hARE-5 (Seq. Id. Nos. 5 & 6)

The full length hARE-5 was cloned by PCR using the hARE5 specific primers 5'-CAGCGCAGGGTGAAGCCTGAGAGC-3' SEQ.ID.NO.: 69 (sense, 5' of initiation codon ATG) and 5'-GGCACCTGCTGTGACCTGTGCAGG-3' SEQ.ID.NO.:70 (antisense, 3' of Stop codon TGA) and human genomic DNA as template. TaqPlus Precision^{™} DNA polymerase (Stratagene) was used for the amplification by the following cycle with step 2 to step 4 repeated 35 times: 96°C, 2 minutes; 96°C, 20 seconds; 58°C, 30 seconds; 72°C, 2 minutes; and 72°C, 10 minutes

A 1.1 Kb PCR fragment of predicated size was isolated and cloned into the pCRII-TOPO^{™} vector (Invitrogen) and completely sequenced (SEQ.ID.NO.:5) using the T7 DNA Sequenase^{™} kit (Amsham).

### h.hARE-4 (Seq. Id. Nos.: 3 & 4)

The full length hARE-4 was cloned by PCR using the hARE-4 specific primers 5'-CTGGTGTGCTCCATGGCATCCC-3' SEQ.ID.NO.:67 (sense, 5' of initiation codon ATG) and 5'-GTAAGCCTCCCAGAACGAGAGG-3' SEQ.ID.NO.: 68 (antisense, 3' of stop codon TGA) and human genomic DNA as template. Taq DNA polymerase (Stratagene) and 5% DMSO was used for the amplification by the following cycle with step 2 to step 3 repeated 35 times: 94°C, 3 minutes; 94°C, 30 seconds; 59°C, 2 minutes; 72°C, 10 minutes

A 1.12 Kb PCR fragment of predicated size was isolated and cloned into the pCRII-TOPO^{™} vector (Invitrogen) and completely sequenced (SEQ.ID.NO.:3) using the T7 DNA Sequenase^{™} kit (Amsham).

### i. hARE-3 (Seq.Id.Nos.: 1 & 2)

The full length hARE-3 was cloned by PCR using the hARE-3 specific primers 5'-gatcaagcttCCATCCTACTGAAACCATGGTC-3' SEQ.ID.NO.:65 (sense, lower case nucleotides represent Hind III overhang, **ATG** as initiation codon) and 5'-gatcagatctCAGTTCCAATAT**TCA**CACCACCGTC-3' SEQ.ID.NO.:66 (antisense, lower case nucleotides represent Xba I overhang, **TCA** as stop codon) and human genomic DNA as template. TaqPlus Precision^{™} DNA polymerase (Stratagene) was used for the amplification by the following cycle with step 2 to step 4 repeated 35 times: 94°C, 3 minutes; 94°C, 1 minute; 55°C, 1 minute; 72°C, 2 minutes; 72°C, 10 minutes.

A 1.3 Kb PCR fragment of predicated size was isolated and digested with Hind III and Xba I, cloned into the pRC/CMV2 vector (Invitrogen) at the Hind III and Xba I sites and completely sequenced (SEQ.ID.NO.:1) using the T7 DNA Sequenase^{™} kit (Amsham).

### j. hRUP3 (Seq. Id. Nos.: 7 & 8)

The full length hRUP3 was cloned by PCR using the hRUP3 specific primers 5'-GTCCTGCCACTTCGAGAC**ATG**G-3' SEQ.ID.NO.:71 (sense, **ATG** as initiation codon) and 5'-GAAACTTCTCTGCCCTTACCGTC-3' SEQ.ID.NO.:72 (antisense, 3' of stop codon TAA) and human genomic DNA as template. TaqPlus Precision^{™} DNA polymerase (Stratagene) was used for the amplification by the following cycle with step 2 to step 4 repeated 35 times: 94°C, 3 minutes; 94°C, 1 minute; 58°C, 1 minute; 72°C, 2 minutes; 72°C, 10 minutes

A 1.0 Kb PCR fragment of predicated size was isolated and cloned into the pCRII-TOPO^{™} vector (Invitrogen) and completely sequenced (SEQ.ID.NO.: 7) using the T7 DNA sequenase kit (Amsham).

### Example 2

### RECEPTOR EXPRESSION

Although a variety of cells are available to the art for the expression of proteins, it is most preferred that mammalian cells be utilized. The primary reason for this is predicated upon practicalities, *i*.*e*., utilization of, *e*.*g*., yeast cells for the expression of a GPCR, while possible, introduces into the protocol a non-mammalian cell which may not (indeed, in the case of yeast, does not) include the receptor-coupling, genetic-mechanism and secretary pathways that have evolved for mammalian systems - thus, results obtained in non-mammalian cells, while of potential use, are not as preferred as that obtained from mammalian cells. Of the mammalian cells, COS-7, 293 and 293T cells are particularly preferred, although the specific mammalian cell utilized can be predicated upon the particular needs of the artisan. The general procedure for expression of the disclosed GPCRs is as follows.

On day one, 1X10⁷ 293T cells per 150mm plate were plated out. On day two, two reaction tubes will be prepared (the proportions to follow for each tube are per plate): tube A will be prepared by mixing 20µg DNA (e.g., pCMV vector; pCMV vector with receptor cDNA, etc.) in 1.2ml serum free DMEM (Irvine Scientific, Irvine, CA); tube B will be prepared by mixing 120µl lipofectamine (Gibco BRL) in 1.2ml serum free DMEM. Tubes A and B are admixed by inversions (several times), followed by incubation at room temperature for 30-45min. The admixture can be referred to as the "transfection mixture". Plated 293T cells are washed with 1XPBS, followed by addition of 10ml serum free DMEM. 2.4ml of the transfection mixture will then be added to the cells, followed by incubation for 4hrs at 37°C/5% CO₂. The transfection mixture was then be removed by aspiration, followed by the addition of 25ml of DMEM/10% Fetal Bovine Serum. Cells will then be incubated at 37°C/5% CO₂. After 72hr incubation, cells can then be harvested and utilized for analysis.

### Example 3

### TISSUE DISTRIBUTION OF THE DISCLOSED HUMAN GPCRS

Several approaches can be used for determination of the tissue distribution of the GPCRs disclosed herein.

### 1. Dot-Blot Analysis

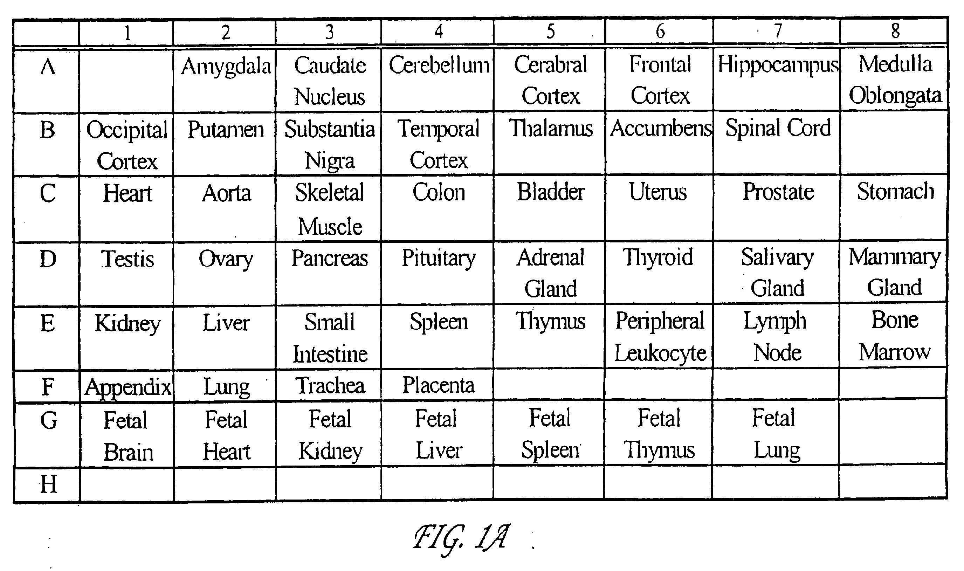

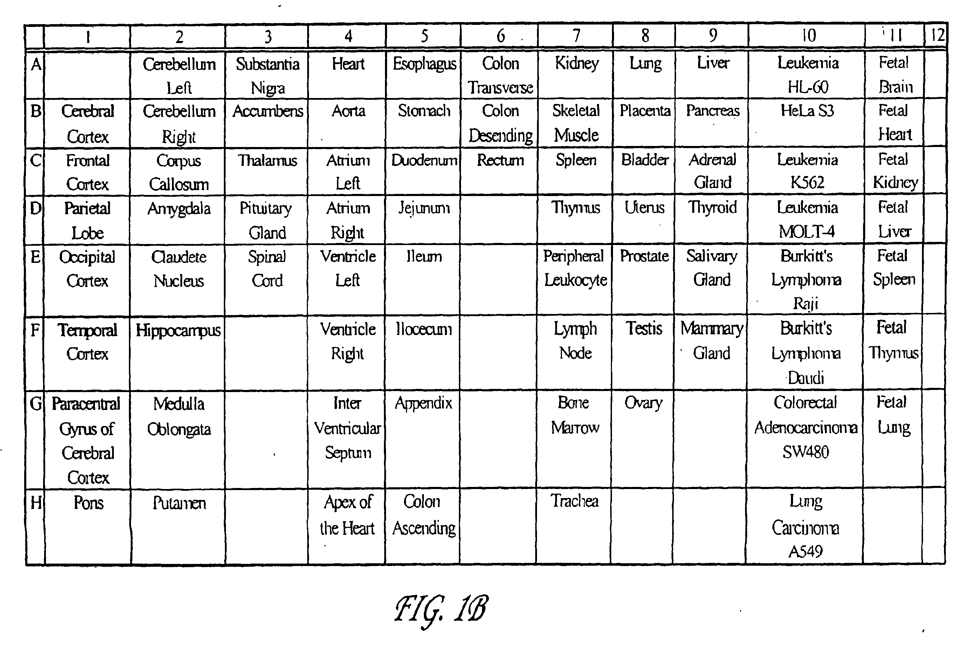

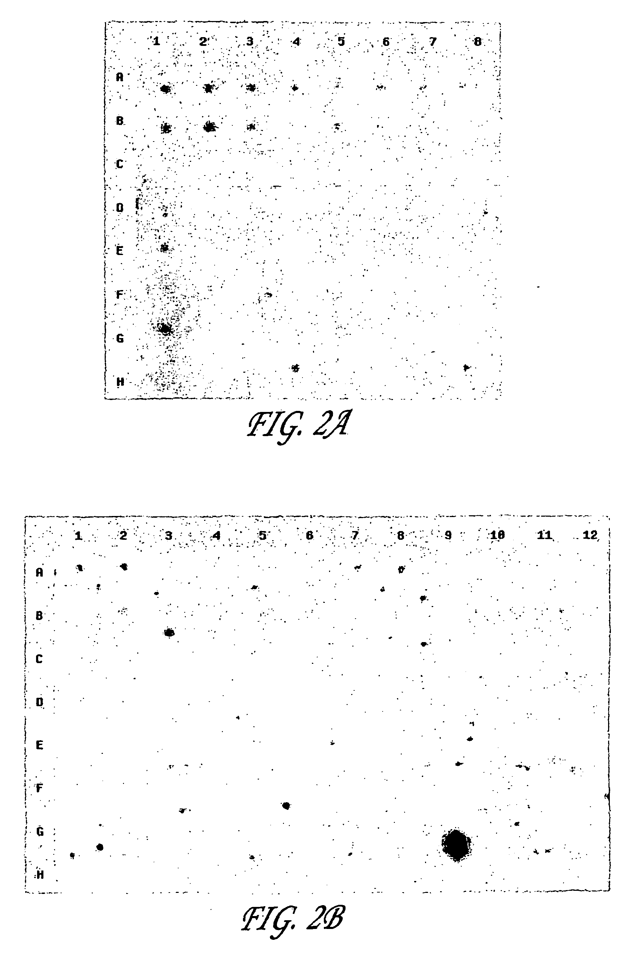

Using a commercially available human-tissue dot-blot format, endogenous orphan GPCRs were probed for a determination of the areas where such receptors are localized. cDNA fragments from the GPCRs of Example 1 (radiolabelled) were (or can be) used as the probe: radiolabeled probe was (or can be) generated using the complete receptor cDNA (excised from the vector) using a Prime-It II^{™} Random Primer Labeling Kit (Stratagene, #300385), according to manufacturer's instructions. A human RNA Master Blot^{™} (Clontech, #7770-1) was hybridized with the endogenous human GPCR radiolabeled probe and washed under stringent conditions according manufacturer's instructions. The blot was exposed to Kodak BioMax^{™} Autoradiography film overnight at -80°C. Results are summarized for several receptors in Table B and C (see Figures 1A and 1B for a grid identifying the various tissues and their locations, respectively). Exemplary dot-blots are provided in Figure 2A and 2B for results derived using hCHN3 and hCHN8, respectively.

**TABLE B**

| **ORPHAN GPCR** | **Tissue Distribution** **(highest levels, relative to other tissues** **in the dot-blot)** |
|---|---|
| hGPCR27 | Fetal brain, Putamen, Pituitary gland, |
| | Caudate nucleus |
| hARE-1 | Spleen, Peripheral leukocytes, Fetal |
| | spleen |
| hPPR1 | Pituitary gland, Heart, salivary gland, |
| | Small intestine, Testis |
| hRUP3 | Pancreas |
| hCHN3 | Fetal brain, Putamen, Occipital cortex |
| hCHN9 | Pancreas, Small intestine, Liver |
| hCHN10 | Kidney, Thryoid |

**TABLE C**

| **ORPHAN GPCR** | **Tissue Distribution** **(highest levels, relative to other tissues** **in the dot-blot)** |
|---|---|
| hARE-3 | Cerebellum left, Cerebellum right, |
| | Testis, Accumbens |
| hGPCR3 | Corpus collusum, Caudate nucleus, Liver, |
| | Heart, Inter-Ventricular Septum |
| hARE-2 | Cerebellum left, Cerebellum right, |
| | Substantia |
| hCHN8 | Cerebellum left, Cerebellum right, |
| | Kidney, Lung |

### 2. RT-PCR

### a. hRUP3

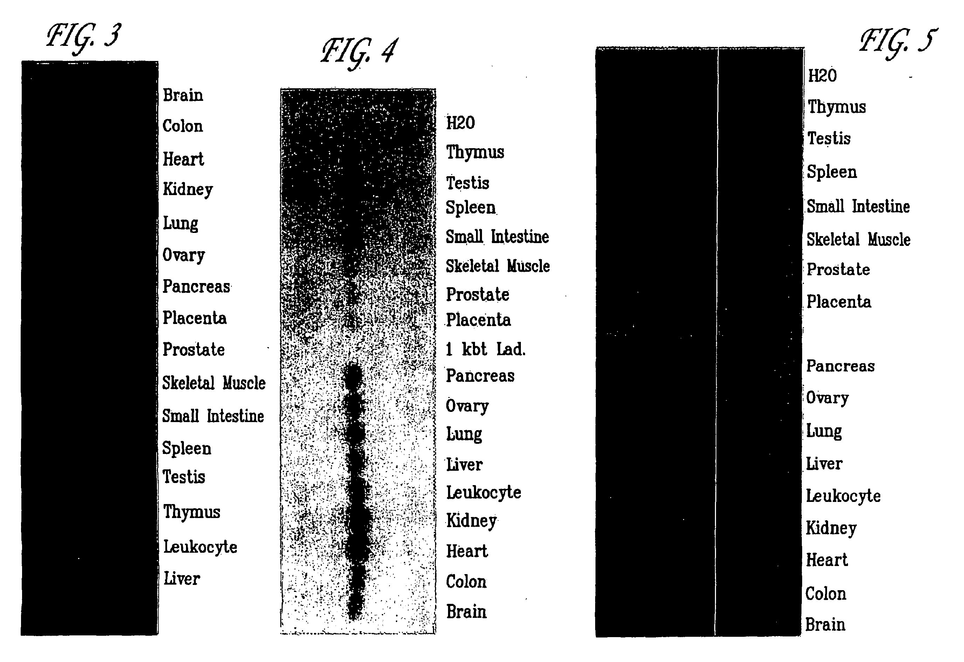

To ascertain the tissue distribution of hRUP3 mRNA, RT-PCR was performed using hRUP3-specific primers and human multiple tissue cDNA panels (MTC, Clontech) as templates. Taq DNA polymerase (Stratagene) was utilized for the PCR reaction, using the following reaction cycles in a 40ul reaction: 94°C for 2 min; 94°C for 15 sec; 55°C for 30 sec; 72°C for 1 min; 72° C, for 10 min. Primers were as follows:
5'-GACAGGTACCTTGCCATCAAG-3' (SEQ.ID.NO.: 61; sense)
5'-CTGCACAATGCCAGTGATAAGG-3' (SEQ.ID.NO.: 62; antisense).
20ul of the reaction was loaded onto a 1% agarose gel; results are set forth in Figure 3.

As is supported by the data of Figure 3, of the 16 human tissues in the cDNA panel utilized (brain, colon, heart, kidney, lung, ovary, pancreas, placenta, prostate, skeleton, small intestine, spleen, testis, thymus leukocyte, and liver) a single hRUP3 band is evident only from the pancreas. Additional comparative analysis of the protein sequence of hRUP3 with other GPCRs suggest that hRUP3 is related to GPCRs having small molecule endogenous ligand such that it is predicted that the endogenous ligand for hRUP3 is a small molecule.

### b. hRUP4

RT-PCR was performed using hRUP4 oligo's 8 and 4 as primers and the human multiple tissue cDNA panels (MTC, Clontech) as templates. Taq DNA polymerase (Stratagene) was used for the amplification in a 40ul reaction by the following cycles: 94°C for 30 seconds, 94°C for 10 seconds, 55°C for 30 seconds, 72°C for 2 minutes, and 72°C for 5 minutes with cycles 2 through 4 repeated 30 times.

20 µl of the reaction were loaded on a 1% agarose gel to analyze the RT-PCR products, and hRUP4 mRNA was found expressed in many human tissues, with the strongest expression in heart and kidney. (*see*, Figure 4). To confirm the authenticity of the PCR fragments, a 300 bp fragment derived from the 5' end of hRUP4 was used as a probe for the Southern Blot analysis. The probe was labeled with ³²P-dCTP using the Prime-It II^{™} Random Primer Labeling Kit (Stratagene) and purified using the ProbeQuant^{™} G-50 micro columns (Amersham). Hybridization was done overnight at 42° C following a 12 hr pre-hybridization. The blot was finally washed at 65°C with 0.1 x SSC. The Southern blot did confirm the PCR fragments as hRUP4.

### c. hRUP5

RT-PCR was performed using the following hRUP5 specific primers:
5'-CTGACTTCTTGTTCCTGGCAGCAGCGG-3' (SEQ.ID.NO.: 63; sense)
5'-AGACCAGCCAGGGCACGCTGAAGAGTG-3' (SEQ.ID.NO.: 64; antisense)
and the human multiple tissue cDNA panels (MTC, Clontech) as templates. Taq DNA polymerase (Stratagene) was used for the amplification in a 40ul reaction by the following cycles: 94°C for 30 sec, 94°C for 10 sec, 62°C for 1.5 min, 72°C for 5 min, and with cycles 2 through 3 repeated 30 times. 20 µl of the reaction were loaded on a 1.5% agarose gel to analyze the RT-PCR products, and hRUP5 mRNA was found expressed only in the peripheral blood leukocytes (*data not shown*)*.*

### d. hRUP6

RT-PCR was applied to confirm the expression and to determine the tissue distribution of hRUP6. Oligonucleotides used, based on an alignment of AC005871 and GPR66 segments, had the following sequences:
5'-CCAACACCAGCATCCATGGCATCAAG-3' (SEQ.2D.NO.: 73; sense),
5'-GGAGAGTCAGCTCTGAAAGAATTCAGG-3' (SEQ.ID.NO.: 74; antisense)
and the human multiple tissue cDNA panels (MTC, Clontech) were used as templates.
PCR was performed using TaqPlus Precision^{™} polymerase (Stratagene; manufacturing instructions will be followed) in a 40ul reaction by the following cycles: 94°C for 30 sec; 94°C 5 sec; 66°C for 40 sec, 72°C for 2.5 min, and 72°C for 7 min. Cycles 2 through 4 were repeated 30 times.
20 ul of the reaction were loaded on a 1.2% agarose gel to analyze the RT-PCR products, and a specific 760bp DNA fragment representing hRUP6 was expressed predominantly in the thymus and with less expression in the heart, kidney, lung, prostate small intestine and testis. (see, Figure 5).

Although a variety of Vectors are available to those in the art, for purposes of utilization for both endogenous and non-endogenous human GPCRs, it is most preferred that the Vector utilized be pCMV. This vector was deposited with the American Type Culture Collection (ATCC) on October 13, 1998 (10801 University Blvd., Manassas, VA 20110-2209 USA) under the provisions of the Budapest Treaty for the International Recognition of the Deposit of Microorganisms for the Purpose of Patent Procedure. The DNA was tested by the ATCC and determined to be. The ATCC has assigned the following deposit number to pCMV: ATCC #203351.

## Claims

1. A method of screening candidate compounds to identify a pharmaceutical agent for a disease or disorder state related to the pancreas, the method comprising:
providing a membrane of a cell expressing a G protein-coupled receptor which is a ligand-independent active version of a receptor having SEQ ID NO: 8, wherein the G protein-coupled receptor couples to a G-protein; and
screening candidate compounds against said G protein-coupled receptor.

2. The method of claim 1, wherein said cell is a eukaryotic host cell.

3. The method of claim 2, wherein said cell is mammalian host cell or a yeast host cell.

4. The method of any one of the preceding claims, wherein said screening comprises detecting cAMP.

5. The method of claim 4, wherein said detection of cAMP comprises ELISA using an anti-cAMP antibody.

6. The method of any one of claims 1-3, wherein said screening comprises using [³⁵S]GTPγS to monitor G-protein coupling to a membrane comprising the G protein-coupled receptor.

7. The method of any one of the preceding claims, wherein the method comprises identifying an agonist of the G protein-coupled receptor.

8. The method of any one of claims 1 to 6, wherein the method comprises identifying a partial agonist of the G protein-coupled receptor.

9. The method of any one of claims 1 to 6, wherein the method comprises identifying an inverse agonist of the G protein-coupled receptor.

10. A method according to any one of claims 7 to 9, wherein the method further comprises confirming that the candidate compound binds to the receptor.

11. A method according to any one of claims 7 to 10, wherein the method further comprises formulating said agonist, partial agonist or inverse agonist as a pharmaceutical.

12. The method of any one of the preceding claims wherein the receptor is an endogenous receptor having SEQ ID NO:8.

13. The method of any one of claims 1 to 11, wherein said receptor is a non-endogenous receptor having a mutation positioned 16 amino acid residues N-terminal from the conserved proline residue within the TM6 domain of SEQ ID NO:8.

## Patentansprüche

1. Verfahren zum Screenen von Kandidatenverbindungen zur Identifikation eines Pharmazeutikums für einen Erkrankungs- oder Leidenszustand im Zusammenhang mit dem Pankreas, wobei das Verfahren Folgendes umfasst:
das Bereitstellen einer Membran einer Zelle, die einen G-Protein-gebundenen Rezeptor exprimiert, der eine ligandenunabhängige aktive Version eines Rezeptors der Seq.-ID Nr. 8 ist, worin der G-Protein-gebundene Rezeptor an ein G-Protein bindet; und
das Screenen von Kandidatenverbindungen gegen den G-Protein-gebundenen Rezeptor.

2. Verfahren nach Anspruch 1, worin die Zelle eine eukaryotische Wirtszelle ist.

3. Verfahren nach Anspruch 2, worin die Zelle eine Säugetier-Wirtszelle oder eine Hefe-Wirtszelle ist.

4. Verfahren nach einem der vorangegangenen Ansprüche, worin das Screenen das Nachweisen von cAMP umfasst.

5. Verfahren nach Anspruch 4, worin das Nachweisen von cAMP einen ELISA unter Einsatz eines Anti-cAMP-Antikörpers umfasst.

6. Verfahren nach einem der Ansprüche 1-3, worin das Screenen den Einsatz von [³⁵S]GTPγS umfasst, um die G-Protein-Bindung an eine Membran zu überwachen, die den G-Protein-gebundenen Rezeptor umfasst.

7. Verfahren nach einem der vorangegangenen Ansprüche, worin das Verfahren das Identifizieren eines Agonisten des G-Protein-gebundenen Rezeptors umfasst.

8. Verfahren nach einem der Ansprüche 1 bis 6, worin das Verfahren das Identifizieren eines partiellen Agonisten des G-Protein-gebundenen Rezeptors umfasst.

9. Verfahren nach einem der Ansprüche 1 bis 6, worin das Verfahren das Identifizieren eines inversen Agonisten des G-Protein-gebundenen Rezeptors umfasst.

10. Verfahren nach einem der Ansprüche 7 bis 9, worin das Verfahren weiters das Bestätigen umfasst, dass die Kandidatenverbindung an den Rezeptor bindet.

11. Verfahren nach einem der Ansprüche 7 bis 10, worin das Verfahren weiters das Formulieren des Agonisten, partiellen Agonisten oder inversen Agonisten als Pharmazeutikum umfasst.

12. Verfahren nach einem der vorangegangenen Ansprüche, worin der Rezeptor ein endogener Rezeptor der Seq.-ID Nr. 8 ist.

13. Verfahren nach einem der Ansprüche 1 bis 11, worin der Rezeptor ein nichtendogener Rezeptor mit einer Mutation ist, die sich 16 Aminosäurereste N-terminal vom konservierten Prolinrest innerhalb der TM6-Domäne von Seq.-ID Nr. 8 befindet.

## Revendications

1. Méthode de criblage de composés candidats pour identifier un agent pharmaceutique pour une maladie ou un état de trouble en rapport avec le pancréas, la méthode consistant à :
prévoir une membrane d'une cellule exprimant un récepteur couplé à la protéine G qui est une version active indépendante du ligand d'un récepteur ayant SEQ ID NO: 8, où le récepteur couplé à la protéine G se couple à une protéine G; et
cribler des composés candidats contre ledit récepteur couplé à la protéine G.

2. Méthode de la revendication 1, où ladite cellule est une cellule hôte eucaryote.

3. Méthode de la revendication 2, où ladite cellule est une cellule hôte mammalienne ou une cellule hôte de levure.

4. Méthode de l'une quelconque des revendications précédentes, où ledit criblage comprend la détection de AMPc.

5. Méthode de la revendication 4, où ladite détection de AMPc comprend ELISA utilisant un anticorps anti-AMPc.

6. Méthode selon l'une quelconque des revendications 1-3, où ledit criblage comprend l'utilisation de [³⁵S]GTP_{Y}S pour surveiller le couplage de la protéine G à une membrane comprenant le récepteur couplé à la protéine G.

7. Méthode de l'une quelconque des revendications précédentes, où la méthode comprend l'identification d'un agoniste du récepteur couplé à la protéine G.

8. Méthode de l'une quelconque des revendications 1 à 6, où la méthode comprend l'identification d'un agoniste partiel du récepteur couplé à la protéine G.

9. Méthode de l'une quelconque des revendications 1 à 6, où la méthode comprend l'identification d'un agoniste inverse du récepteur couplé à la protéine G.

10. Méthode selon l'une quelconque des revendications 7 à 9, où la méthode comprend de plus la confirmation que le composé candidat se lie au récepteur.

11. Méthode selon l'une quelconque des revendications 7 à 10, où la méthode comprend de plus la formulation dudit agoniste, agoniste partiel ou agoniste inverse en tant que produit pharmaceutique.

12. Méthode de l'une quelconque des revendications précédentes où le récepteur est un récepteur endogène ayant SEQ ID NO:8.

13. Méthode de l'une quelconque des revendications 1 à 11, où ledit récepteur est un récepteur non endogène ayant une mutation positionnée au 16 résidus d'acides aminés N-terminaux à partir du résidu conservé de proline dans le domaine de TM6 de SEQ ID NO:8.


### SEQUENCE LISTING

<110> Arena Pharmaceuticals, Inc.
<120> Human Orphan G Protein-Coupled Receptors
<130> JEC/FP6461016
<140> Divisional of EP 05003040.2
   <141> 1999 10 13
<150> EP 05003040.2
   <151> 1999 10 13
<150> EP 99972682.1
   <151> 1999 10 13
<150> PCT/US99/23687
   <151> 1999 10 13
<150> 60/109,213
   <151> 1998 11 20
<150> 60/120,416
   <151> 1999 02 16
<150> 60/121,852
   <151> 1999 02 26
<150> 60/123,946
   <151> 1999 03 12
<150> 60/123,949
   <151> 1999 03 12
<150> 60/136,436
   <151> 1999 05 28
<150> 60/136,437
   <151> 1999 05 28
<150> 60/136,439
   <151> 1999 05 28
<150> 60/136,567
   <151> 1999 05 28
<150> 60/137,127
   <151> 1999 05 28
<150> 60/137,131
   <151> 1999 05 28
<150> 60/141,448
<151> 1999 06 29
<150> 60/156,653
<151> 1999 09 29
<150> 60/156,333
   <151> 1999 09 29
<150> 60/156,555
   <151> 1999 09 29
<150> 60/156,634
   <151> 1999 09 29
<150> 60/157,280
   <151> 1999 10 01
<150> 60/157,294
   <151> 1999 10 01
<150> 60/157,281
   <151> 1999 10 01
<150> 60/157,293
   <151> 1999 10 01
<150> 60/157,282
   <151> 1999 10 01
<150> 09/417,044
   <151> 1999 10 12
<150> 09/416,760
   <151> 1999 10 12
<160> 74
<170> PatentIn Ver. 2.1
<210> 1
   <211> 1260
   <212> DNA
   <213> Homo sapiens
<400> 1
   atggtcttct cggcagtgtt gactgcgttc cataccggga catccaacac aacatttgtc 60
   gtgtatgaaa acacctacat gaatattaca ctccctccac cattccagca tcctgacctc 120
   agtccattgc ttagatatag ttttgaaacc atggctccca ctggtttgag ttccttgacc 180
   gtgaatagta cagctgtgcc cacaacacca gcagcattta agagcctaaa cttgcctctt 240
   cagatcaccc tttctgctat aatgatattc attctgtttg tgtcttttct tgggaacttg 300
   gttgtttgcc tcatggttta ccaaaaagct gccatgaggt ctgcaattaa catcctcctt 360
   gccagcctag cttttgcaga catgttgctt gcagtgctga acatgccctt tgccctggta 420
   actattctta ctacccgatg gatttttggg aaattcttct gtagggtatc tgctatgttt 480
   ttctggttat ttgtgataga aggagtagcc atcctgctca tcattagcat agataggttc 540
   cttattatag tccagaggca ggataagcta aacccatata gagctaaggt tctgattgca 600
   gtttcttggg caacttcctt ttgtgtagct tttcctttag ccgtaggaaa ccccgacctg 660
   cagatacctt cccgagctcc ccagtgtgtg tttgggtaca caaccaatcc aggctaccag 720
   gcttatgtga ttttgatttc tctcatttct ttcttcatac ccttcctggt aatactgtac 780
   tcatttatgg gcatactcaa cacccttcgg cacaatgcct tgaggatcca tagctaccct 840
   gaaggtatat gcctcagcca ggccagcaaa ctgggtctca tgagtctgca gagacctttc 900
   cagatgagca ttgacatggg ctttaaaaca cgtgccttca ccactatttt gattctcttt 960
   gctgtcttca ttgtctgctg ggccccattc accacttaca gccttgtggc aacattcagt 1020
   aagcactttt actatcagca caactttttt gagattagca cctggctact gtggctctgc 1080
   tacctcaagt ctgcattgaa tccgctgatc tactactgga ggattaagaa attccatgat 1140
   gcttgcctgg acatgatgcc taagtccttc aagtttttgc cgcagctccc tggtcacaca 1200
   aagcgacgga tacgtcctag tgctgtctat gtgtgtgggg aacatcggac ggtggtgtga 1260
<210> 2
   <211> 419
   <212> PRT
   <213> Homo sapiens
<400> 2
   Met Val Phe Ser Ala Val Leu Thr Ala Phe His Thr Gly Thr Ser Asn
   1               5                   10                  15
   Thr Thr Phe Val Val Tyr Glu Asn Thr Tyr Met Asn Ile Thr Leu Pro
               20                  25                  30
   Pro Pro Phe Gln His Pro Asp Leu Ser Pro Leu Leu Arg Tyr Ser Phe
           35                  40                  45
   Glu Thr Met Ala Pro Thr Gly Leu Ser Ser Leu Thr Val Asn Ser Thr
       50                  55                  60
   Ala Val Pro Thr Thr Pro Ala Ala Phe Lys Ser Leu Asn Leu Pro Leu
   65                  70                  75                  80
   Gln Ile Thr Leu Ser Ala Ile Met Ile Phe Ile Leu Phe Val Ser Phe
                   85                  90                  95
   Leu Gly Asn Leu Val Val Cys Leu Met Val Tyr Gln Lys Ala Ala Met
               100                 105                 110
   Arg Ser Ala Ile Asn Ile Leu Leu Ala Ser Leu Ala Phe Ala Asp Met
           115                 120                 125
   Leu Leu Ala Val Leu Asn Met Pro Phe Ala Leu Val Thr Ile Leu Thr
       130                 135                 140
   Thr Arg Trp Ile Phe Gly Lys Phe Phe Cys Arg Val Ser Ala Met Phe
   145                 150                 155                 160
   Phe Trp Leu Phe Val Ile Glu Gly Val Ala Ile Leu Leu Ile Ile Ser
                   165                 170                 175
   Ile Asp Arg Phe Leu Ile Ile Val Gln Arg Gln Asp Lys Leu Asn Pro
               180                 185                 190
   Tyr Arg Ala Lys Val Leu Ile Ala Val Ser Trp Ala Thr Ser Phe Cys
           195                 200                 205
   Val Ala Phe Pro Leu Ala Val Gly Asn Pro Asp Leu Gln Ile Pro Ser
       210                 215                 220
   Arg Ala Pro Gln Cys Val Phe Gly Tyr Thr Thr Asn Pro Gly Tyr Gln
   225                 230                 235                 240
   Ala Tyr Val Ile Leu Ile Ser Leu Ile Ser Phe Phe Ile Pro Phe Leu
                   245                 250                 255
   Val Ile Leu Tyr Ser Phe Met Gly Ile Leu Asn Thr Leu Arg His Asn
               260                 265                 270
   Ala Leu Arg Ile His Ser Tyr Pro Glu Gly Ile Cys Leu Ser Gln Ala
           275                 280                 285
   Ser Lys Leu Gly Leu Met Ser Leu Gln Arg Pro Phe Gln Met Ser Ile
       290                 295                 300
   Asp Met Gly Phe Lys Thr Arg Ala Phe Thr Thr Ile Leu Ile Leu Phe
   305                 310                 315                 320
   Ala Val Phe Ile Val Cys Trp Ala Pro Phe Thr Thr Tyr Ser Leu Val
                   325                 330                 335
   Ala Thr Phe Ser Lys His Phe Tyr Tyr Gln His Asn Phe Phe Glu Ile
               340                 345                 350
   Ser Thr Trp Leu Leu Trp Leu Cys Tyr Leu Lys Ser Ala Leu Asn Pro
           355                 360                 365
   Leu Ile Tyr Tyr Trp Arg Ile Lys Lys Phe His Asp Ala Cys Leu Asp
       370                 375                 380
   Met Met Pro Lys Ser Phe Lys Phe Leu Pro Gln Leu Pro Gly His Thr
   385                 390                 395                 400
   Lys Arg Arg Ile Arg Pro Ser Ala Val Tyr Val Cys Gly Glu His Arg
                   405                 410                 415
   Thr Val Val
<210> 3
   <211> 1119
   <212> DNA
   <213> Homo sapiens
<400> 3
   atgttagcca acagctcctc aaccaacagt tctgttctcc cgtgtcctga ctaccgacct 60
   acccaccgcc tgcacttggt ggtctacagc ttggtgctgg ctgccgggct ccccctcaac 120
   gcgctagccc tctgggtctt cctgcgcgcg ctgcgcgtgc actcggtggt gagcgtgtac 180
   atgtgtaacc tggcggccag cgacctgctc ttcaccctct cgctgcccgt tcgtctctcc 240
   tactacgcac tgcaccactg gcccttcccc gacctcctgt gccagacgac gggcgccatc 300
   ttccagatga acatgtacgg cagctgcatc ttcctgatgc tcatcaagct ggaccgctac 360
   gccgccatcg tgcacccgct gcgactgcgc cacctgcggc ggccccgcgt ggcgcggctg 420
   ctctgcctgg gcgtgtgggc gctcatcctg gtgtttgccg tgcccgccgc ccgcgtgcac 480
   aggccctcgc gttgccgcta ccgggacctc gaggtgcgcc tatgcttcga gagcttcagc 540
   gacgagctgt ggaaaggcag gctgctgccc ctcgtgctgc tggccgaggc gctgggcttc 600
   ctgctgcccc tggcggcggt ggtctactcg tcgggccgag tcttctggac gctggcgcgc 660
   cccgacgcca cgcagagcca gcggcggcgg aagaccgtgc gcctcctgct ggctaacctc 720
   gtcatcttcc tgctgtgctt cgtgccctac aacagcacgc tggcggtcta cgggctgctg 780
   cggagcaagc tggtggcggc cagcgtgcct gcccgcgatc gcgtgcgcgg ggtgctgatg 840
   gtgatggtgc tgctggccgg cgccaactgc gtgctggacc cgctggtgta ctactttagc 900
   gccgagggct tccgcaacac cctgcgcggc ctgggcactc cgcaccgggc caggacctcg 960
   gccaccaacg ggacgcgggc ggcgctcgcg caatccgaaa ggtccgccgt caccaccgac 1020
   gccaccaggc cggatgccgc cagtcagggg ctgctccgac cctccgactc ccactctctg 1080
   tcttccttca cacagtgtcc ccaggattcc gccctctga 1119
<210> 4
   <211> 372
   <212> PRT
   <213> Homo sapiens
<400> 4
<210> 5
   <211> 1107
   <212> DNA
   <213> Homo sapiens
<400> 5
   atggccaact ccacagggct gaacgcctca gaagtcgcag gctcgttggg gttgatcctg 60
   gcagctgtcg tggaggtggg ggcactgctg ggcaacggcg cgctgctggt cgtggtgctg 120
   cgcacgccgg gactgcgcga cgcgctctac ctggcgcacc tgtgcgtcgt ggacctgctg 180
   gcggccgcct ccatcatgcc gctgggcctg ctggccgcac cgccgcccgg gctgggccgc 240
   gtgcgcctgg gccccgcgcc atgccgcgcc gctcgcttcc tctccgccgc tctgctgccg 300
   gcctgcacgc tcggggtggc cgcacttggc ctggcacgct accgcctcat cgtgcacccg 360
   ctgcggccag gctcgcggcc gccgcctgtg ctcgtgctca ccgccgtgtg ggccgcggcg 420
   ggactgctgg gcgcgctctc cctgctcggc ccgccgcccg caccgccccc tgctcctgct 480
   cgctgctcgg tcctggctgg gggcctcggg cccttccggc cgctctgggc cctgctggcc 540
   ttcgcgctgc ccgccctcct gctgctcggc gcctacggcg gcatcttcgt ggtggcgcgt 600
   cgcgctgccc tgaggccccc acggccggcg cgcgggtccc gactccgctc ggactctctg 660
   gatagccgcc tttccatctt gccgccgctc cggcctcgcc tgcccggggg caaggcggcc 720
   ctggccccag cgctggccgt gggccaattt gcagcctgct ggctgcctta tggctgcgcg 780
   tgcctggcgc ccgcagcgcg ggccgcggaa gccgaagcgg ctgtcacctg ggtcgcctac 840
   tcggccttcg cggctcaccc cttcctgtac gggctgctgc agcgccccgt gcgcttggca 900
   ctgggccgcc tctctcgccg tgcactgcct ggacctgtgc gggcctgcac tccgcaagcc 960
   tggcacccgc gggcactctt gcaatgcctc cagagacccc cagagggccc tgccgtaggc 1020
   ccttctgagg ctccagaaca gacccccgag ttggcaggag ggcggagccc cgcataccag 1080
   gggccacctg agagttctct ctcctga                                     1107
<210> 6
   <211> 368
   <212> PRT
   <213> Homo sapiens
<400> 6
   Met Ala Asn Ser Thr Gly Leu Asn Ala Ser Glu Val Ala Gly Ser Leu
   1               5                   10                  15
   Gly Leu Ile Leu Ala Ala Val Val Glu Val Gly Ala Leu Leu Gly Asn
               20                  25                  30
   Gly Ala Leu Leu Val Val Val Leu Arg Thr Pro Gly Leu Arg Asp Ala
           35                  40                  45
   Leu Tyr Leu Ala His Leu Cys Val Val Asp Leu Leu Ala Ala Ala Ser
       50                  55                  60
   Ile Met Pro Leu Gly Leu Leu Ala Ala Pro Pro Pro Gly Leu Gly Arg
   65                  70                  75                  80
   Val Arg Leu Gly Pro Ala Pro Cys Arg Ala Ala Arg Phe Leu Ser Ala
                   85                  90                  95
   Ala Leu Leu Pro Ala Cys Thr Leu Gly Val Ala Ala Leu Gly Leu Ala
               100                 105                 110
   Arg Tyr Arg Leu Ile Val His Pro Leu Arg Pro Gly Ser Arg Pro Pro
           115                 120                 125
   Pro Val Leu Val Leu Thr Ala Val Trp Ala Ala Ala Gly Leu Leu Gly
       130                 135                 140
   Ala Leu Ser Leu Leu Gly Pro Pro Pro Ala Pro Pro Pro Ala Pro Ala
   145                 150                 155                 160
   Arg Cys Ser Val Leu Ala Gly Gly Leu Gly Pro Phe Arg Pro Leu Trp
                   165                 170                 175
   Ala Leu Leu Ala Phe Ala Leu Pro Ala Leu Leu Leu Leu Gly Ala Tyr
               180                 185                 190
   Gly Gly Ile Phe Val Val Ala Arg Arg Ala Ala Leu Arg Pro Pro Arg
           195                 200                 205
   Pro Ala Arg Gly Ser Arg Leu Arg Ser Asp Ser Leu Asp Ser Arg Leu
       210                 215                 220
   Ser Ile Leu Pro Pro Leu Arg Pro Arg Leu Pro Gly Gly Lys Ala Ala
   225                 230                 235                 240
   Leu Ala Pro Ala Leu Ala Val Gly Gln Phe Ala Ala Cys Trp Leu Pro
                   245                 250                 255
   Tyr Gly Cys Ala Cys Leu Ala Pro Ala Ala Arg Ala Ala Glu Ala Glu
               260                 265                 270
   Ala Ala Val Thr Trp Val Ala Tyr Ser Ala Phe Ala Ala His Pro Phe
           275                 280                 285
   Leu Tyr Gly Leu Leu Gln Arg Pro Val Arg Leu Ala Leu Gly Arg Leu
       290                 295                 300
   Ser Arg Arg Ala Leu Pro Gly Pro Val Arg Ala Cys Thr Pro Gln Ala
   305                 310                 315                 320
   Trp His Pro Arg Ala Leu Leu Gln Cys Leu Gln Arg Pro Pro Glu Gly
                   325                 330                 335
   Pro Ala Val Gly Pro Ser Glu Ala Pro Glu Gln Thr Pro Glu Leu Ala
               340                 345                 350
   Gly Gly Arg Ser Pro Ala Tyr Gln Gly Pro Pro Glu Ser Ser Leu Ser
           355                 360                 365
<210> 7
   <211> 1008
   <212> DNA
   <213> Homo sapiens
<400> 7
   atggaatcat ctttctcatt tggagtgatc cttgctgtcc tggcctccct catcattgct 60
   actaacacac tagtggctgt ggctgtgctg ctgttgatcc acaagaatga tggtgtcagt 120
   ctctgcttca ccttgaatct ggctgtggct gacaccttga ttggtgtggc catctctggc 180
   ctactcacag accagctctc cagcccttct cggcccacac agaagaccct gtgcagcctg 240
   cggatggcat ttgtcacttc ctccgcagct gcctctgtcc tcacggtcat gctgatcacc 300
   tttgacaggt accttgccat caagcagccc ttccgctact tgaagatcat gagtgggttc 360
   gtggccgggg cctgcattgc cgggctgtgg ttagtgtctt acctcattgg cttcctccca 420
   ctcggaatcc ccatgttcca gcagactgcc tacaaagggc agtgcagctt ctttgctgta 480
   tttcaccctc acttcgtgct gaccctctcc tgcgttggct tcttcccagc catgctcctc 540
   tttgtcttct tctactgcga catgctcaag attgcctcca tgcacagcca gcagattcga 600
   aagatggaac atgcaggagc catggctgga ggttatcgat ccccacggac tcccagcgac 660
   ttcaaagctc tccgtactgt gtctgttctc attgggagct ttgctctatc ctggaccccc 720
   ttccttatca ctggcattgt gcaggtggcc tgccaggagt gtcacctcta cctagtgctg 780
   gaacggtacc tgtggctgct cggcgtgggc aactccctgc tcaacccact catctatgcc 840
   tattggcaga aggaggtgcg actgcagctc taccacatgg ccctaggagt gaagaaggtg 900
   ctcacctcat tcctcctctt tctctcggcc aggaattgtg gcccagagag gcccagggaa 960
   agttcctgtc acatcgtcac tatctccagc tcagagtttg atggctaa             1008
<210> 8
   <211> 335
   <212> PRT
   <213> Homo sapiens
<400> 8
   Met Glu Ser Ser Phe Ser Phe Gly Val Ile Leu Ala Val Leu Ala Ser
   1               5                   10                  15
   Leu Ile Ile Ala Thr Asn Thr Leu Val Ala Val Ala Val Leu Leu Leu
               20                  25                  30
   Ile His Lys Asn Asp Gly Val Ser Leu Cys Phe Thr Leu Asn Leu Ala
           35                  40                  45
   Val Ala Asp Thr Leu Ile Gly Val Ala Ile Ser Gly Leu Leu Thr Asp
       50                  55                  60
   Gln Leu Ser Ser Pro Ser Arg Pro Thr Gln Lys Thr Leu Cys Ser Leu
   65                  70                  75                  80
   Arg Met Ala Phe Val Thr Ser Ser Ala Ala Ala Ser Val Leu Thr Val
                   85                  90                  95
   Met Leu Ile Thr Phe Asp Arg Tyr Leu Ala Ile Lys Gln Pro Phe Arg
               100                 105                 110
   Tyr Leu Lys Ile Met Ser Gly Phe Val Ala Gly Ala Cys Ile Ala Gly
           115                 120                 125
   Leu Trp Leu Val Ser Tyr Leu Ile Gly Phe Leu Pro Leu Gly Ile Pro
       130                 135                 140
   Met Phe Gln Gln Thr Ala Tyr Lys Gly Gln Cys Ser Phe Phe Ala Val
   145                 150                 155                 160
   Phe His Pro His Phe Val Leu Thr Leu Ser Cys Val Gly Phe Phe Pro
                   165                 170                 175
   Ala Met Leu Leu Phe Val Phe Phe Tyr Cys Asp Met Leu Lys Ile Ala
               180                 185                 190
   Ser Met His Ser Gln Gln Ile Arg Lys Met Glu His Ala Gly Ala Met
           195                 200                 205
   Ala Gly Gly Tyr Arg Ser Pro Arg Thr Pro Ser Asp Phe Lys Ala Leu
       210                 215                 220
   Arg Thr Val Ser Val Leu Ile Gly Ser Phe Ala Leu Ser Trp Thr Pro
   225                 230                 235                 240
   Phe Leu Ile Thr Gly Ile Val Gln Val Ala Cys Gln Glu Cys His Leu
                   245                 250                 255
   Tyr Leu Val Leu Glu Arg Tyr Leu Trp Leu Leu Gly Val Gly Asn Ser
               260                 265                 270
   Leu Leu Asn Pro Leu Ile Tyr Ala Tyr Trp Gln Lys Glu Val Arg Leu
           275                 280                 285
   Gln Leu Tyr His Met Ala Leu Gly Val Lys Lys Val Leu Thr Ser Phe
       290                 295                 300
   Leu Leu Phe Leu Ser Ala Arg Asn Cys Gly Pro Glu Arg Pro Arg Glu
   305                 310                 315                 320
   Ser Ser Cys His Ile Val Thr Ile Ser Ser Ser Glu Phe Asp Gly
                   325                 330                 335
<210> 9
   <211> 1413
   <212> DNA
   <213> Homo sapiens
<400> 9
   atggacacta ccatggaagc tgacctgggt gccactggcc acaggccccg cacagagctt 60
   gatgatgagg actcctaccc ccaaggtggc tgggacacgg tcttcctggt ggccctgctg 120
   ctccttgggc tgccagccaa tgggttgatg gcgtggctgg ccggctccca ggcccggcat 180
   ggagctggca cgcgtctggc gctgctcctg ctcagcctgg ccctctctga cttcttgttc 240
   ctggcagcag cggccttcca gatcctagag atccggcatg ggggacactg gccgctgggg 300
   acagctgcct gccgcttcta ctacttccta tggggcgtgt cctactcctc cggcctcttc 360
   ctgctggccg ccctcagcct cgaccgctgc ctgctggcgc tgtgcccaca ctggtaccct 420
   gggcaccgcc cagtccgcct gcccctctgg gtctgcgccg gtgtctgggt gctggccaca 480
   ctcttcagcg tgccctggct ggtcttcccc gaggctgccg tctggtggta cgacctggtc 540
   atctgcctgg acttctggga cagcgaggag ctgtcgctga ggatgctgga ggtcctgggg 600
   ggcttcctgc ctttcctcct gctgctcgtc tgccacgtgc tcacccaggc cacagcctgt 660
   cgcacctgcc accgccaaca gcagcccgca gcctgccggg gcttcgcccg tgtggccagg 720
   accattctgt cagcctatgt ggtcctgagg ctgccctacc agctggccca gctgctctac 780
   ctggccttcc tgtgggacgt ctactctggc tacctgctct gggaggccct ggtctactcc 840
   gactacctga tcctactcaa cagctgcctc agccccttcc tctgcctcat ggccagtgcc 900
   gacctccgga ccctgctgcg ctccgtgctc tcgtccttcg cggcagctct ctgcgaggag 960
   cggccgggca gcttcacgcc cactgagcca cagacccagc tagattctga gggtccaact 1020
   ctgccagagc cgatggcaga ggcccagtca cagatggatc ctgtggccca gcctcaggtg 1080
   aaccccacac tccagccacg atcggatccc acagctcagc cacagctgaa ccctacggcc 1140
   cagccacagt cggatcccac agcccagcca cagctgaacc tcatggccca gccacagtca 1200
   gattctgtgg cccagccaca ggcagacact aacgtccaga cccctgcacc tgctgccagt 1260
   tctgtgccca gtccctgtga tgaagcttcc ccaaccccat cctcgcatcc taccccaggg 1320
   gcccttgagg acccagccac acctcctgcc tctgaaggag aaagccccag cagcaccccg 1380
   ccagaggcgg ccccgggcgc aggccccacg tga                               1413
<210> 10
   <211> 468
   <212> PRT
   <213> Homo sapiens
<400> 10
   Met Asp Thr Thr Met Glu Ala Asp Leu Gly Ala Thr Gly His Arg Pro
   1               5                   10                  15
   Arg Thr Glu Leu Asp Asp Glu Asp Ser Tyr Pro Gln Gly Gly Trp Asp
               20                  25                  30
   Thr Val Phe Leu Val Ala Leu Leu Leu Leu Gly Leu Pro Ala Asn Gly
           35                  40                  45
   Leu Met Ala Trp Leu Ala Gly Ser Gln Ala Arg His Gly Ala Gly Thr
       50                  55                  60
   Arg Leu Ala Leu Leu Leu Leu Ser Leu Ala Leu Ser Asp Phe Leu Phe
   65                  70                  75                  80
   Leu Ala Ala Ala Ala Phe Gln Ile Leu Glu Ile Arg His Gly Gly His
                   85                  90                  95
   Trp Pro Leu Gly Thr Ala Ala Cys Arg Phe Tyr Tyr Phe Leu Trp Gly
               100                 105                 110
   Val Ser Tyr Ser Ser Gly Leu Phe Leu Leu Ala Ala Leu Ser Leu Asp
           115                 120                 125
   Arg Cys Leu Leu Ala Leu Cys Pro His Trp Tyr Pro Gly His Arg Pro
       130                 135                 140
   Val Arg Leu Pro Leu Trp Val Cys Ala Gly Val Trp Val Leu Ala Thr
   145                 150                 155                 160
   Leu Phe Ser Val Pro Trp Leu Val Phe Pro Glu Ala Ala Val Trp Trp
                   165                 170                 175
   Tyr Asp Leu Val Ile Cys Leu Asp Phe Trp Asp Ser Glu Glu Leu Ser
               180                 185                 190
   Leu Arg Met Leu Glu Val Leu Gly Gly Phe Leu Pro Phe Leu Leu Leu
           195                 200                 205
   Leu Val Cys His Val Leu Thr Gln Ala Thr Arg Thr Cys His Arg Gln
       210                 215                 220
   Gln Gln Pro Ala Ala Cys Arg Gly Phe Ala Arg Val Ala Arg Thr Ile
   225                 230                 235                 240
   Leu Ser Ala Tyr Val Val Leu Arg Leu Pro Tyr Gln Leu Ala Gln Leu
                   245                 250                 255
   Leu Tyr Leu Ala Phe Leu Trp Asp Val Tyr Ser Gly Tyr Leu Leu Trp
               260                 265                 270
   Glu Ala Leu Val Tyr Ser Asp Tyr Leu Ile Leu Leu Asn Ser Cys Leu
           275                 280                 285
   Ser Pro Phe Leu Cys Leu Met Ala Ser Ala Asp Leu Arg Thr Leu Leu
       290                 295                 300
   Arg Ser Val Leu Ser Ser Phe Ala Ala Ala Leu Cys Glu Glu Arg Pro
   305                 310                 315                 320
   Gly Ser Phe Thr Pro Thr Glu Pro Gln Thr Gln Leu Asp Ser Glu Gly
                   325                 330                 335
   Pro Thr Leu Pro Glu Pro Met Ala Glu Ala Gln Ser Gln Met Asp Pro
               340                 345                 350
   Val Ala Gln Pro Gln Val Asn Pro Thr Leu Gln Pro Arg Ser Asp Pro
           355                 360                 365
   Thr Ala Gln Pro Gln Leu Asn Pro Thr Ala Gln Pro Gln Ser Asp Pro
       370                 375                 380
   Thr Ala Gln Pro Gln Leu Asn Leu Met Ala Gln Pro Gln Ser Asp Ser
   385                 390                 395                 400
   Val Ala Gln Pro Gln Ala Asp Thr Asn Val Gln Thr Pro Ala Pro Ala
                   405                 410                 415
   Ala Ser Ser Val Pro Ser Pro Cys Asp Glu Ala Ser Pro Thr Pro Ser
               420                 425                 430
   Ser His Pro Thr Pro Gly Ala Leu Glu Asp Pro Ala Thr Pro Pro Ala
           435                 440                 445
   Ser Glu Gly Glu Ser Pro Ser Ser Thr Pro Pro Glu Ala Ala Pro Gly
       450                 455                 460
   Ala Gly Pro Thr
   465
<210> 11
   <211> 1248
   <212> DNA
   <213> Homo sapiens
<400> 11
   atgtcaggga tggaaaaact tcagaatgct tcctggatct accagcagaa actagaagat 60
   ccattccaga aacacctgaa cagcaccgag gagtatctgg ccttcctctg cggacctcgg 120
   cgcagccact tcttcctccc cgtgtctgtg gtgtatgtgc caatttttgt ggtgggggtc 180
   attggcaatg tcctggtgtg cctggtgatt ctgcagcacc aggctatgaa gacgcccacc 240
   aactactacc tcttcagcct ggcggtctct gacctcctgg tcctgctcct tggaatgccc 300
   ctggaggtct atgagatgtg gcgcaactac cctttcttgt tcgggcccgt gggctgctac 360
   ttcaagacgg ccctctttga gaccgtgtgc ttcgcctcca tcctcagcat caccaccgtc 420
   agcgtggagc gctacgtggc catcctacac ccgttccgcg ccaaactgca gagcacccgg 480
   cgccgggccc tcaggatcct cggcatcgtc tggggcttct ccgtgctctt ctccctgccc 540
   aacaccagca tccatggcat caagttccac tacttcccca atgggtccct ggtcccaggt 600
   tcggccacct gtacggtcat caagcccatg tggatctaca atttcatcat ccaggtcacc 660
   tccttcctat tctacctcct ccccatgact gtcatcagtg tcctctacta cctcatggca 720
   ctcagactaa agaaagacaa atctcttgag gcagatgaag ggaatgcaaa tattcaaaga 780
   ccctgcagaa aatcagtcaa caagatgctg tttgtcttgg tcttagtgtt tgctatctgt 840
   tgggccccgt tccacattga ccgactcttc ttcagctttg tggaggagtg gagtgaatcc 900
   ctggctgctg tgttcaacct cgtccatgtg gtgtcaggtg tcttcttcta cctgagctca 960
   gctgtcaacc ccattatcta taacctactg tctcgccgct tccaggcagc attccagaat 1020
   gtgatctctt ctttccacaa acagtggcac tcccagcatg acccacagtt gccacctgcc 1080
   cagcggaaca tcttcctgac agaatgccac tttgtggagc tgaccgaaga tataggtccc 1140
   caattcccat gtcagtcatc catgcacaac tctcacctcc caacagccct ctctagtgaa 1200
   cagatgtcaa gaacaaacta tcaaagcttc cactttaaca aaacctga             1248
<210> 12
   <211> 415
   <212> PRT
   <213> Homo sapiens
<400> 12
<210> 13
   <211> 1173
   <212> DNA
   <213> Homo sapiens
<400> 13
   atgccagata ctaatagcac aatcaattta tcactaagca ctcgtgttac tttagcattt 60
   tttatgtcct tagtagcttt tgctataatg ctaggaaatg ctttggtcat tttagctttt 120
   gtggtggaca aaaaccttag acatcgaagt agttattttt ttcttaactt ggccatctct 180
   gacttctttg tgggtgtgat ctccattcct ttgtacatcc ctcacacgct gttcgaatgg 240
   gattttggaa aggaaatctg tgtattttgg ctcactactg actatctgtt atgtacagca 300
   tctgtatata acattgtcct catcagctat gatcgatacc tgtcagtctc aaatgctgtg 360
   tcttatagaa ctcaacatac tggggtcttg aagattgtta ctctgatggt ggccgtttgg 420
   gtgctggcct tcttagtgaa tgggccaatg attctagttt cagagtcttg gaaggatgaa 480
   ggtagtgaat gtgaacctgg atttttttcg gaatggtaca tccttgccat cacatcattc 540
   ttggaattcg tgatcccagt catcttagtc gcttatttca acatgaatat ttattggagc 600
   ctgtggaagc gtgatcatct cagtaggtgc caaagccatc ctggactgac tgctgtctct 660
   tccaacatct gtggacactc attcagaggt agactatctt caaggagatc tctttctgca 720
   tcgacagaag ttcctgcatc ctttcattca gagagacaga ggagaaagag tagtctcatg 780
   ttttcctcaa gaaccaagat gaatagcaat acaattgctt ccaaaatggg ttccttctcc 840
   caatcagatt ctgtagctct tcaccaaagg gaacatgttg aactgcttag agccaggaga 900
   ttagccaagt cactggccat tctcttaggg gtttttgctg tttgctgggc tccatattct 960
   ctgttcacaa ttgtcctttc attttattcc tcagcaacag gtcctaaatc agtttggtat 1020
   agaattgcat tttggcttca gtggttcaat tcctttgtca atcctctttt gtatccattg 1080
   tgtcacaagc gctttcaaaa ggctttcttg aaaatatttt gtataaaaaa gcaacctcta 1140
   ccatcacaac acagtcggtc agtatcttct taa                              1173
<210> 14
   <211> 390
   <212> PRT
   <213> Homo sapiens
<400> 14
   Met Pro Asp Thr Asn Ser Thr Ile Asn Leu Ser Leu Ser Thr Arg Val
   1               5                   10                  15
   Thr Leu Ala Phe Phe Met Ser Leu Val Ala Phe Ala Ile Met Leu Gly
               20                  25                  30
   Asn Ala Leu Val Ile Leu Ala Phe Val Val Asp Lys Asn Leu Arg His
           35                  40                  45
   Arg Ser Ser Tyr Phe Phe Leu Asn Leu Ala Ile Ser Asp Phe Phe Val
       50                  55                  60
   Gly Val Ile Ser Ile Pro Leu Tyr Ile Pro His Thr Leu Phe Glu Trp
   65                  70                  75                  80
   Asp Phe Gly Lys Glu Ile Cys Val Phe Trp Leu Thr Thr Asp Tyr Leu
                   85                  90                  95
   Leu Cys Thr Ala Ser Val Tyr Asn Ile Val Leu Ile Ser Tyr Asp Arg
               100                 105                 110
   Tyr Leu Ser Val Ser Asn Ala Val Ser Tyr Arg Thr Gln His Thr Gly
           115                 120                 125
   Val Leu Lys Ile Val Thr Leu Met Val Ala Val Trp Val Leu Ala Phe
       130                 135                 140
   Leu Val Asn Gly Pro Met Ile Leu Val Ser Glu Ser Trp Lys Asp Glu
   145                 150                 155                 160
   Gly Ser Glu Cys Glu Pro Gly Phe Phe Ser Glu Trp Tyr Ile Leu Ala
                   165                 170                 175
   Ile Thr Ser Phe Leu Glu Phe Val Ile Pro Val Ile Leu Val Ala Tyr
               180                 185                 190
   Phe Asn Met Asn Ile Tyr Trp Ser Leu Trp Lys Arg Asp His Leu Ser
           195                 200                 205
   Arg Cys Gln Ser His Pro Gly Leu Thr Ala Val Ser Ser Asn Ile Cys
       210                 215                 220
   Gly His Ser Phe Arg Gly Arg Leu Ser Ser Arg Arg Ser Leu Ser Ala
   225                 230                 235                 240
   Ser Thr Glu Val Pro Ala Ser Phe His Ser Glu Arg Gln Arg Arg Lys
                   245                 250                 255
   Ser Ser Leu Met Phe Ser Ser Arg Thr Lys Met Asn Ser Asn Thr Ile
               260                 265                 270
   Ala Ser Lys Met Gly Ser Phe Ser Gln Ser Asp Ser Val Ala Leu His
           275                 280                 285
   Gln Arg Glu His Val Glu Leu Leu Arg Ala Arg Arg Leu Ala Lys Ser
       290                 295                 300
   Leu Ala Ile Leu Leu Gly Val Phe Ala Val Cys Trp Ala Pro Tyr Ser
   305                 310                 315                 320
   Leu Phe Thr Ile Val Leu Ser Phe Tyr Ser Ser Ala Thr Gly Pro Lys
                   325                 330                 335
   Ser Val Trp Tyr Arg Ile Ala Phe Trp Leu Gln Trp Phe Asn Ser Phe
               340                 345                 350
   Val Asn Pro Leu Leu Tyr Pro Leu Cys His Lys Arg Phe Gln Lys Ala
           355                 360                 365
   Phe Leu Lys Ile Phe Cys Ile Lys Lys Gln Pro Leu Pro Ser Gln His
       370                 375                 380
   Ser Arg Ser Val Ser Ser
   385                 390
<210> 15
   <211> 1128
   <212> DNA
   <213> Homo sapiens
<400> 15
   atggcgaacg cgagcgagcc gggtggcagc ggcggcggcg aggcggccgc cctgggcctc 60
   aagctggcca cgctcagcct gctgctgtgc gtgagcctag cgggcaacgt gctgttcgcg 120
   ctgctgatcg tgcgggagcg cagcctgcac cgcgccccgt actacctgct gctcgacctg 180
   tgcctggccg acgggctgcg cgcgctcgcc tgcctcccgg ccgtcatgct ggcggcgcgg 240
   cgtgcggcgg ccgcggcggg ggcgccgccg ggcgcgctgg gctgcaagct gctcgccttc 300
   ctggccgcgc tcttctgctt ccacgccgcc ttcctgctgc tgggcgtggg cgtcacccgc 360
   tacctggcca tcgcgcacca ccgcttctat gcagagcgcc tggccggctg gccgtgcgcc 420
   gccatgctgg tgtgcgccgc ctgggcgctg gcgctggccg cggccttccc gccagtgctg 480
   gacggcggtg gcgacgacga ggacgcgccg tgcgccctgg agcagcggcc cgacggcgcc 540
   cccggcgcgc tgggcttcct gctgctgctg gccgtggtgg tgggcgccac gcacctcgtc 600
   tacctccgcc tgctcttctt catccacgac cgccgcaaga tgcggcccgc gcgcctggtg 660
   cccgccgtca gccacgactg gaccttccac ggcccgggcg ccaccggcca ggcggccgcc 720
   aactggacgg cgggcttcgg ccgcgggccc acgccgcccg cgcttgtggg catccggccc 780
   gcagggccgg gccgcggcgc gcgccgcctc ctcgtgctgg aagaattcaa gacggagaag 840
   aggctgtgca agatgttcta cgccgtcacg ctgctcttcc tgctcctctg gggcccctac 900
   gtcgtggcca gctacctgcg ggtcctggtg cggcccggcg ccgtccccca ggcctacctg 960
   acggcctccg tgtggctgac cttcgcgcag gccggcatca accccgtcgt gtgcttcctc 1020
   ttcaacaggg agctgaggga ctgcttcagg gcccagttcc cctgctgcca gagcccccgg 1080
   accacccagg cgacccatcc ctgcgacctg aaaggcattg gtttatga             1128
<210> 16
   <211> 375
   <212> PRT
   <213> Homo sapiens
<400> 16
   Met Ala Asn Ala Ser Glu Pro Gly Gly Ser Gly Gly Gly Glu Ala Ala
   1               5                   10                  15
   Ala Leu Gly Leu Lys Leu Ala Thr Leu Ser Leu Leu Leu Cys Val Ser
               20                  25                  30
   Leu Ala Gly Asn Val Leu Phe Ala Leu Leu Ile Val Arg Glu Arg Ser
           35                  40                  45
   Leu His Arg Ala Pro Tyr Tyr Leu Leu Leu Asp Leu Cys Leu Ala Asp
       50                  55                  60
   Gly Leu Arg Ala Leu Ala Cys Leu Pro Ala Val Met Leu Ala Ala Arg
   65                  70                  75                  80
   Arg Ala Ala Ala Ala Ala Gly Ala Pro Pro Gly Ala Leu Gly Cys Lys
                   85                  90                  95
   Leu Leu Ala Phe Leu Ala Ala Leu Phe Cys Phe His Ala Ala Phe Leu
               100                 105                 110
   Leu Leu Gly Val Gly Val Thr Arg Tyr Leu Ala Ile Ala His His Arg
           115                 120                 125
   Phe Tyr Ala Glu Arg Leu Ala Gly Trp Pro Cys Ala Ala Met Leu Val
       130                 135                 140
   Cys Ala Ala Trp Ala Leu Ala Leu Ala Ala Ala Phe Pro Pro Val Leu
   145                 150                 155                 160
   Asp Gly Gly Gly Asp Asp Glu Asp Ala Pro Cys Ala Leu Glu Gln Arg
                   165                 170                 175
   Pro Asp Gly Ala Pro Gly Ala Leu Gly Phe Leu Leu Leu Leu Ala Val
               180                 185                 190
   Val Val Gly Ala Thr His Leu Val Tyr Leu Arg Leu Leu Phe Phe Ile
           195                 200                 205
   His Asp Arg Arg Lys Met Arg Pro Ala Arg Leu Val Pro Ala Val Ser
       210                 215                 220
   His Asp Trp Thr Phe His Gly Pro Gly Ala Thr Gly Gln Ala Ala Ala
   225                 230                 235                 240
   Asn Trp Thr Ala Gly Phe Gly Arg Gly Pro Thr Pro Pro Ala Leu Val
                   245                 250                 255
   Gly Ile Arg Pro Ala Gly Pro Gly Arg Gly Ala Arg Arg Leu Leu Val
               260                 265                 270
   Leu Glu Glu Phe Lys Thr Glu Lys Arg Leu Cys Lys Met Phe Tyr Ala
           275                 280                 285
   Val Thr Leu Leu Phe Leu Leu Leu Trp Gly Pro Tyr Val Val Ala Ser
       290                 295                 300
   Tyr Leu Arg Val Leu Val Arg Pro Gly Ala Val Pro Gln Ala Tyr Leu
   305                 310                 315                 320
   Thr Ala Ser Val Trp Leu Thr Phe Ala Gln Ala Gly Ile Asn Pro Val
                   325                 330                 335
   Val Cys Phe Leu Phe Asn Arg Glu Leu Arg Asp Cys Phe Arg Ala Gln
               340                 345                 350
   Phe Pro Cys Cys Gln Ser Pro Arg Thr Thr Gln Ala Thr His Pro Cys
           355                 360                 365
   Asp Leu Lys Gly Ile Gly Leu
       370                 375
<210> 17
   <211> 1002
   <212> DNA
   <213> Homo sapiens
<400> 17
   atgaacacca cagtgatgca aggcttcaac agatctgagc ggtgccccag agacactcgg 60
   atagtacagc tggtattccc agccctctac acagtggttt tcttgaccgg catcctgctg 120
   aatactttgg ctctgtgggt gtttgttcac atccccagct cctccacctt catcatctac 180
   ctcaaaaaca ctttggtggc cgacttgata atgacactca tgcttccttt caaaatcctc 240
   tctgactcac acctggcacc ctggcagctc agagcttttg tgtgtcgttt ttcttcggtg 300
   atattttatg agaccatgta tgtgggcatc gtgctgttag ggctcatagc ctttgacaga 360
   ttcctcaaga tcatcagacc tttgagaaat atttttctaa aaaaacctgt ttttgcaaaa 420
   acggtctcaa tcttcatctg gttctttttg ttcttcatct ccctgccaaa tacgatcttg 480
   agcaacaagg aagcaacacc atcgtctgtg aaaaagtgtg cttccttaaa ggggcctctg 540
   gggctgaaat ggcatcaaat ggtaaataac atatgccagt ttatttctgg gactgttttt 600
   atcctaatgc ttgtgtttta tgtggttatt gcaaaaaaag tatatgattc ttatagaaag 660
   tccaaaagta aggacagaaa aaacaacaaa aagctggaag gcaaagtatt tgttgtcgtg 720
   gctgtcttct ttgtgtgttt tgctccattt cattttgcca gagttccata tactcacagt 780
   caaaccaaca ataagactga ctgtagactg caaaatcaac tgtttattgc taaagaaaca 840
   actctctttt tggcagcaac taacatttgt atggatccct taatatacat attcttatgt 900
   aaaaaattca cagaaaagct accatgtatg caagggagaa agaccacagc atcaagccaa 960
   gaaaatcata gcagtcagac agacaacata accttaggct ga                   1002
<210> 18
   <211> 333
   <212> PRT
   <213> Homo sapiens
<400> 18
   Met Asn Thr Thr Val Met Gln Gly Phe Asn Arg Ser Glu Arg Cys Pro
    1               5                  10                  15
   Arg Asp Thr Arg Ile Val Gln Leu Val Phe Pro Ala Leu Tyr Thr Val
               20                  25                  30
   Val Phe Leu Thr Gly Ile Leu Leu Asn Thr Leu Ala Leu Trp Val Phe
           35                  40                  45
   Val His Ile Pro Ser Ser Ser Thr Phe Ile Ile Tyr Leu Lys Asn Thr
       50                  55                  60
   Leu Val Ala Asp Leu Ile Met Thr Leu Met Leu Pro Phe Lys Ile Leu
   65                  70                  75                  80
   Ser Asp Ser His Leu Ala Pro Trp Gln Leu Arg Ala Phe Val Cys Arg
                   85                  90                  95
   Phe Ser Ser Val Ile Phe Tyr Glu Thr Met Tyr Val Gly Ile Val Leu
               100                 105                 110
   Leu Gly Leu Ile Ala Phe Asp Arg Phe Leu Lys Ile Ile Arg Pro Leu
           115                 120                 125
   Arg Asn Ile Phe Leu Lys Lys Pro Val Phe Ala Lys Thr Val Ser Ile
       130                 135                 140
   Phe Ile Trp Phe Phe Leu Phe Phe Ile Ser Leu Pro Asn Thr Ile Leu
   145                 150                 155                 160
   Ser Asn Lys Glu Ala Thr Pro Ser Ser Val Lys Lys Cys Ala Ser Leu
                   165                 170                 175
   Lys Gly Pro Leu Gly Leu Lys Trp His Gln Met Val Asn Asn Ile Cys
               180                 185                 190
   Gln Phe Ile Phe Trp Thr Val Phe Ile Leu Met Leu Val Phe Tyr Val
           195                 200                 205
   Val Ile Ala Lys Lys Val Tyr Asp Ser Tyr Arg Lys Ser Lys Ser Lys
       210                 215                 220
   Asp Arg Lys Asn Asn Lys Lys Leu Glu Gly Lys Val Phe Val Val Val
   225                 230                 235                 240
   Ala Val Phe Phe Val Cys Phe Ala Pro Phe His Phe Ala Arg Val Pro
                   245                 250                 255
   Tyr Thr His Ser Gln Thr Asn Asn Lys Thr Asp Cys Arg Leu Gln Asn
               260                 265                 270
   Gln Leu Phe Ile Ala Lys Glu Thr Thr Leu Phe Leu Ala Ala Thr Asn
           275                 280                 285
   Ile Cys Met Asp Pro Leu Ile Tyr Ile Phe Leu Cys Lys Lys Phe Thr
       290                 295                 300
   Glu Lys Leu Pro Cys Met Gln Gly Arg Lys Thr Thr Ala Ser Ser Gln
   305                 310                 315                 320
   Glu Asn His Ser Ser Gln Thr Asp Asn Ile Thr Leu Gly
                   325                 330
<210> 19
   <211> 1122
   <212> DNA
   <213> Homo sapiens
<400> 19
   atggccaaca ctaccggaga gcctgaggag gtgagcggcg ctctgtcccc accgtccgca 60
   tcagcttatg tgaagctggt actgctggga ctgattatgt gcgtgagcct ggcgggtaac 120
   gccatcttgt ccctgctggt gctcaaggag cgtgccctgc acaaggctcc ttactacttc 180
   ctgctggacc tgtgcctggc cgatggcata cgctctgccg tctgcttccc ctttgtgctg 240
   gcttctgtgc gccacggctc ttcatggacc ttcagtgcac tcagctgcaa gattgtggcc 300
   tttatggccg tgctcttttg cttccatgcg gccttcatgc tgttctgcat cagcgtcacc 360
   cgctacatgg ccatcgccca ccaccgcttc tacgccaagc gcatgacact ctggacatgc 420
   gcggctgtca tctgcatggc ctggaccctg tctgtggcca tggccttccc acctgtcttt 480
   gacgtgggca cctacaagtt tattcgggag gaggaccagt gcatctttga gcatcgctac 540
   ttcaaggcca atgacacgct gggcttcatg cttatgttgg ctgtgctcat ggcagctacc 600
   catgctgtct acggcaagct gctcctcttc gagtatcgtc accgcaagat gaagccagtg 660
   cagatggtgc cagccatcag ccagaactgg acattccatg gtcccggggc caccggccag 720
   gctgctgcca actggatcgc cggctttggc cgtgggccca tgccaccaac cctgctgggt 780
   atccggcaga atgggcatgc agccagccgg cggctactgg gcatggacga ggtcaagggt 840
   gaaaagcagc tgggccgcat gttctacgcg atcacactgc tctttctgct cctctggtca 900
   ccctacatcg tggcctgcta ctggcgagtg tttgtgaaag cctgtgctgt gccccaccgc 960
   tacctggcca ctgctgtttg gatgagcttc gcccaggctg ccgtcaaccc aattgtctgc 1020
   ttcctgctca acaaggacct caagaagtgc ctgaccactc acgccccctg ctggggcaca 1080
   ggaggtgccc cggctcccag agaaccctac tgtgtcatgt ga                    1122
<210> 20
   <211> 373
   <212> PRT
   <213> Homo sapiens
<400> 20
   Met Ala Asn Thr Thr Gly Glu Pro Glu Glu Val Ser Gly Ala Leu Ser
    1               5                  10                  15
   Pro Pro Ser Ala Ser Ala Tyr Val Lys Leu Val Leu Leu Gly Leu Ile
               20                  25                  30
   Met Cys Val Ser Leu Ala Gly Asn Ala Ile Leu Ser Leu Leu Val Leu
           35                  40                  45
   Lys Glu Arg Ala Leu His Lys Ala Pro Tyr Tyr Phe Leu Leu Asp Leu
       50                  55                  60
   Cys Leu Ala Asp Gly Ile Arg Ser Ala Val Cys Phe Pro Phe Val Leu
   65                  70                  75                  80
   Ala Ser Val Arg His Gly Ser Ser Trp Thr Phe Ser Ala Leu Ser Cys
                   85                  90                  95
   Lys Ile Val Ala Phe Met Ala Val Leu Phe Cys Phe His Ala Ala Phe
               100                 105                 110
   Met Leu Phe Cys Ile Ser Val Thr Arg Tyr Met Ala Ile Ala His His
           115                 120                 125
   Arg Phe Tyr Ala Lys Arg Met Thr Leu Trp Thr Cys Ala Ala Val Ile
       130                 135                 140
   Cys Met Ala Trp Thr Leu Ser Val Ala Met Ala Phe Pro Pro Val Phe
   145                 150                 155                 160
   Asp Val Gly Thr Tyr Lys Phe Ile Arg Glu Glu Asp Gln Cys Ile Phe
                   165                 170                 175
   Glu His Arg Tyr Phe Lys Ala Asn Asp Thr Leu Gly Phe Met Leu Met
               180                 185                 190
   Leu Ala Val Leu Met Ala Ala Thr His Ala Val Tyr Gly Lys Leu Leu
           195                 200                 205
   Leu Phe Glu Tyr Arg His Arg Lys Met Lys Pro Val Gln Met Val Pro
       210                 215                 220
   Ala Ile Ser Gln Asn Trp Thr Phe His Gly Pro Gly Ala Thr Gly Gln
   225                 230                 235                 240
   Ala Ala Ala Asn Trp Ile Ala Gly Phe Gly Arg Gly Pro Met Pro Pro
                   245                 250                 255
   Thr Leu Leu Gly Ile Arg Gln Asn Gly His Ala Ala Ser Arg Arg Leu
               260                 265                 270
   Leu Gly Met Asp Glu Val Lys Gly Glu Lys Gln Leu Gly Arg Met Phe
           275                 280                 285
   Tyr Ala Ile Thr Leu Leu Phe Leu Leu Leu Trp Ser Pro Tyr Ile Val
       290                 295                 300
   Ala Cys Tyr Trp Arg Val Phe Val Lys Ala Cys Ala Val Pro His Arg
   305                 310                 315                 320
   Tyr Leu Ala Thr Ala Val Trp Met Ser Phe Ala Gln Ala Ala Val Asn
                   325                 330                 335
   Pro Ile Val Cys Phe Leu Leu Asn Lys Asp Leu Lys Lys Cys Leu Thr
               340                 345                 350
   Thr His Ala Pro Cys Trp Gly Thr Gly Gly Ala Pro Ala Pro Arg Glu
           355                 360                 365
   Pro Tyr Cys Val Met
       370
<210> 21
   <211> 1053
   <212> DNA
   <213> Homo sapiens
<400> 21
   atggctttgg aacagaacca gtcaacagat tattattatg aggaaaatga aatgaatggc 60
   acttatgact acagtcaata tgaattgatc tgtatcaaag aagatgtcag agaatttgca 120
   aaagttttcc tccctgtatt cctcacaata gctttcgtca ttggacttgc aggcaattcc 180
   atggtagtgg caatttatgc ctattacaag aaacagagaa ccaaaacaga tgtgtacatc 240
   ctgaatttgg ctgtagcaga tttactcctt ctattcactc tgcctttttg ggctgttaat 300
   gcagttcatg ggtgggtttt agggaaaata atgtgcaaaa taacttcagc cttgtacaca 360
   ctaaactttg tctctggaat gcagtttctg gcttgcatca gcatagacag atatgtggca 420
   gtaactaatg tccccagcca atcaggagtg ggaaaaccat gctggatcat ctgtttctgt 480
   gtctggatgg ctgccatctt gctgagcata ccccagctgg ttttttatac agtaaatgac 540
   aatgctaggt gcattcccat tttcccccgc tacctaggaa catcaatgaa agcattgatt 600
   caaatgctag agatctgcat tggatttgta gtaccctttc ttattatggg ggtgtgctac 660
   tttatcacgg caaggacact catgaagatg ccaaacatta aaatatctcg acccctaaaa 720
   gttctgctca cagtcgttat agttttcatt gtcactcaac tgccttataa cattgtcaag 780
   ttctgccgag ccatagacat catctactcc ctgatcacca gctgcaacat gagcaaacgc 840
   atggacatcg ccatccaagt cacagaaagc attgcactct ttcacagctg cctcaaccca 900
   atcctttatg tttttatggg agcatctttc aaaaactacg ttatgaaagt ggccaagaaa 960
   tatgggtcct ggagaagaca gagacaaagt gtggaggagt ttccttttga ttctgagggt 1020
   cctacagagc caaccagtac ttttagcatt taa 1053
<210> 22
   <211> 350
   <212> PRT
   <213> Homo sapiens
<400> 22
<210> 23
   <211> 1116
   <212> DNA
   <213> Homo sapiens
<400> 23
   atgccaggaa acgccacccc agtgaccacc actgccccgt gggcctccct gggcctctcc 60
   gccaagacct gcaacaacgt gtccttcgaa gagagcagga tagtcctggt cgtggtgtac 120
   agcgcggtgt gcacgctggg ggtgccggcc aactgcctga ctgcgtggct ggcgctgctg 180
   caggtactgc agggcaacgt gctggccgtc tacctgctct gcctggcact ctgcgaactg 240
   ctgtacacag gcacgctgcc actctgggtc atctatatcc gcaaccagca ccgctggacc 300
   ctaggcctgc tggcctcgaa ggtgaccgcc tacatcttct tctgcaacat ctacgtcagc 360
   atcctcttcc tgtgctgcat ctcctgcgac cgcttcgtgg ccgtggtgta cgcgctggag 420
   agtcggggcc gccgccgccg gaggaccgcc atcctcatct ccgcctgcat cttcatcctc 480
   gtcgggatcg ttcactaccc ggtgttccag acggaagaca aggagacctg ctttgacatg 540
   ctgcagatgg acagcaggat tgccgggtac tactacgcca ggttcaccgt tggctttgcc 600
   atccctctct ccatcatcgc cttcaccaac caccggattt tcaggagcat caagcagagc 660
   atgggcttaa gcgctgccca gaaggccaag gtgaagcact cggccatcgc ggtggttgtc 720
   atcttcctag tctgcttcgc cccgtaccac ctggttctcc tcgtcaaagc cgctgccttt 780
   tcctactaca gaggagacag gaacgccatg tgcggcttgg aggaaaggct gtacacagcc 840
   tctgtggtgt ttctgtgcct gtccacggtg aacggcgtgg ctgaccccat tatctacgtg 900
   ctggccacgg accattcccg ccaagaagtg tccagaatcc ataaggggtg gaaagagtgg 960
   tccatgaaga cagacgtcac caggctcacc cacagcaggg acaccgagga gctgcagtcg 1020
   cccgtggccc ttgcagacca ctacaccttc tccaggcccg tgcacccacc agggtcacca 1080
   tgccctgcaa agaggctgat tgaggagtcc tgctga                            1116
<210> 24
   <211> 371
   <212> PRT
   <213> Homo sapiens
<400> 24
   Met Pro Gly Asn Ala Thr Pro Val Thr Thr Thr Ala Pro Trp Ala Ser
    1               5                  10                  15
   Leu Gly Leu Ser Ala Lys Thr Cys Asn Asn Val Ser Phe Glu Glu Ser
               20                  25                  30
   Arg Ile Val Leu Val Val Val Tyr Ser Ala Val Cys Thr Leu Gly Val
           35                  40                  45
   Pro Ala Asn Cys Leu Thr Ala Trp Leu Ala Leu Leu Gln Val Leu Gln
       50                  55                  60
   Gly Asn Val Leu Ala Val Tyr Leu Leu Cys Leu Ala Leu Cys Glu Leu
   65                  70                  75                  80
   Leu Tyr Thr Gly Thr Leu Pro Leu Trp Val Ile Tyr Ile Arg Asn Gln
                   85                  90                  95
   His Arg Trp Thr Leu Gly Leu Leu Ala Ser Lys Val Thr Ala Tyr Ile
               100                 105                 110
   Phe Phe Cys Asn Ile Tyr Val Ser Ile Leu Phe Leu Cys Cys Ile Ser
           115                 120                 125
   Cys Asp Arg Phe Val Ala Val Val Tyr Ala Leu Glu Ser Arg Gly Arg
       130                 135                 140
   Arg Arg Arg Arg Thr Ala Ile Leu Ile Ser Ala Cys Ile Phe Ile Leu
   145                 150                 155                 160
   Val Gly Ile Val His Tyr Pro Val Phe Gln Thr Glu Asp Lys Glu Thr
                   165                 170                 175
   Cys Phe Asp Met Leu Gln Met Asp Ser Arg Ile Ala Gly Tyr Tyr Tyr
               180                 185                 190
   Ala Arg Phe Thr Val Gly Phe Ala Ile Pro Leu Ser Ile Ile Ala Phe
           195                 200                 205
   Thr Asn His Arg Ile Phe Arg Ser Ile Lys Gln Ser Met Gly Leu Ser
       210                 215                 220
   Ala Ala Gln Lys Ala Lys Val Lys His Ser Ala Ile Ala Val Val Val
   225                 230                 235                 240
   Ile Phe Leu Val Cys Phe Ala Pro Tyr His Leu Val Leu Leu Val Lys
   245 250 255
   Ala Ala Ala Phe Ser Tyr Tyr Arg Gly Asp Arg Asn Ala Met Cys Gly
   260 265 270
   Leu Glu Glu Arg Leu Tyr Thr Ala Ser Val Val Phe Leu Cys Leu Ser
   275 280 285
   Thr Val Asn Gly Val Ala Asp Pro Ile Ile Tyr Val Leu Ala Thr Asp
   290 295 300
   His Ser Arg Gln Glu Val Ser Arg Ile His Lys Gly Trp Lys Glu Trp
   305 310 315 320
   Ser Met Lys Thr Asp Val Thr Arg Leu Thr His Ser Arg Asp Thr Glu
   325 330 335
   Glu Leu Gln Ser Pro Val Ala Leu Ala Asp His Tyr Thr Phe Ser Arg
   340 345 350
   Pro Val His Pro Pro Gly Ser Pro Cys Pro Ala Lys Arg Leu Ile Glu
   355 360 365
   Glu Ser Cys
   370
<210> 25
   <211> 1113
   <212> DNA
   <213> Homo sapiens
<400> 25
   atggcgaact atagccatgc agctgacaac attttgcaaa atctctcgcc tctaacagcc 60
   tttctgaaac tgacttcctt gggtttcata ataggagtca gcgtggtggg caacctcctg 120
   atctccattt tgctagtgaa agataagacc ttgcatagag caccttacta cttcctgttg 180
   gatctttgct gttcagatat cctcagatct gcaatttgtt tcccatttgt gttcaactct 240
   gtcaaaaatg gctctacctg gacttatggg actctgactt gcaaagtgat tgcctttctg 300
   ggggttttgt cctgtttcca cactgctttc atgctcttct gcatcagtgt caccagatac 360
   ttagctatcg cccatcaccg cttctataca aagaggctga ccttttggac gtgtctggct 420
   gtgatctgta tggtgtggac tctgtctgtg gccatggcat ttcccccggt tttagacgtg 480
   ggcacttact cattcattag ggaggaagat caatgcacct tccaacaccg ctccttcagg 540
   gctaatgatt ccttaggatt tatgctgctt cttgctctca tcctcctagc cacacagctt 600
   gtctacctca agctgatatt tttcgtccac gatcgaagaa aaatgaagcc agtccagttt 660
   gtagcagcag tcagccagaa ctggactttt catggtcctg gagccagtgg ccaggcagct 720
   gccaattggc tagcaggatt tggaaggggt cccacaccac ccaccttgct gggcatcagg 780
   caaaatgcaa acaccacagg cagaagaagg ctattggtct tagacgagtt caaaatggag 840
   aaaagaatca gcagaatgtt ctatataatg acttttctgt ttctaacctt gtggggcccc 900
   tacctggtgg cctgttattg gagagttttt gcaagagggc ctgtagtacc agggggattt 960
   ctaacagctg ctgtctggat gagttttgcc caagcaggaa tcaatccttt tgtctgcatt 1020
   ttctcaaaca gggagctgag gcgctgtttc agcacaaccc ttctttactg cagaaaatcc 1080
   aggttaccaa gggaacctta ctgtgttata tga 1113
<210> 26
   <211> 370
   <212> PRT
   <213> Homo sapiens
<400> 26
   Met Ala Asn Tyr Ser His Ala Ala Asp Asn Ile Leu Gln Asn Leu Ser
   1               5                   10                  15
   Pro Leu Thr Ala Phe Leu Lys Leu Thr Ser Leu Gly Phe Ile Ile Gly
               20                  25                  30
   Val Ser Val Val Gly Asn Leu Leu Ile Ser Ile Leu Leu Val Lys Asp
           35                  40                  45
   Lys Thr Leu His Arg Ala Pro Tyr Tyr Phe Leu Leu Asp Leu Cys Cys
       50                  55                  60
   Ser Asp Ile Leu Arg Ser Ala Ile Cys Phe Pro Phe Val Phe Asn Ser
   65                  70                  75                  80
   Val Lys Asn Gly Ser Thr Trp Thr Tyr Gly Thr Leu Thr Cys Lys Val
                   85                  90                  95
   Ile Ala Phe Leu Gly Val Leu Ser Cys Phe His Thr Ala Phe Met Leu
               100                 105                 110
   Phe Cys Ile Ser Val Thr Arg Tyr Leu Ala Ile Ala His His Arg Phe
           115                 120                 125
   Tyr Thr Lys Arg Leu Thr Phe Trp Thr Cys Leu Ala Val Ile Cys Met
       130                 135                 140
   Val Trp Thr Leu Ser Val Ala Met Ala Phe Pro Pro Val Leu Asp Val
   145                 150                 155                 160
   Gly Thr Tyr Ser Phe Ile Arg Glu Glu Asp Gln Cys Thr Phe Gln His
                   165                 170                 175
   Arg Ser Phe Arg Ala Asn Asp Ser Leu Gly Phe Met Leu Leu Leu Ala
               180                 185                 190
   Leu Ile Leu Leu Ala Thr Gln Leu Val Tyr Leu Lys Leu Ile Phe Phe
           195                 200                 205
   Val His Asp Arg Arg Lys Met Lys Pro Val Gln Phe Val Ala Ala Val
       210                 215                 220
   Ser Gln Asn Trp Thr Phe His Gly Pro Gly Ala Ser Gly Gln Ala Ala
   225                 230                 235                 240
   Ala Asn Trp Leu Ala Gly Phe Gly Arg Gly Pro Thr Pro Pro Thr Leu
                   245                 250                 255
   Leu Gly Ile Arg Gln Asn Ala Asn Thr Thr Gly Arg Arg Arg Leu Leu
               260                 265                 270
   Val Leu Asp Glu Phe Lys Met Glu Lys Arg Ile Ser Arg Met Phe Tyr
           275                 280                 285
   Ile Met Thr Phe Leu Phe Leu Thr Leu Trp Gly Pro Tyr Leu Val Ala
       290                 295                 300
   Cys Tyr Trp Arg Val Phe Ala Arg Gly Pro Val Val Pro Gly Gly Phe
   305 310 315 320
   Leu Thr Ala Ala Val Trp Met Ser Phe Ala Gln Ala Gly Ile Asn Pro
   325 330 335
   Phe Val Cys Ile Phe Ser Asn Arg Glu Leu Arg Arg Cys Phe Ser Thr
   340 345 350
   Thr Leu Leu Tyr Cys Arg Lys Ser Arg Leu Pro Arg Glu Pro Tyr Cys
   355 360 365
   Val Ile
   370
<210> 27
   <211> 1080
   <212> DNA
   <213> Homo sapiens
<400> 27
   atgcaggtcc cgaacagcac cggcccggac aacgcgacgc tgcagatgct gcggaacccg 60
   gcgatcgcgg tggccctgcc cgtggtgtac tcgctggtgg cggcggtcag catcccgggc 120
   aacctcttct ctctgtgggt gctgtgccgg cgcatggggc ccagatcccc gtcggtcatc 180
   ttcatgatca acctgagcgt cacggacctg atgctggcca gcgtgttgcc tttccaaatc 240
   tactaccatt gcaaccgcca ccactgggta ttcggggtgc tgctttgcaa cgtggtgacc 300
   gtggcctttt acgcaaacat gtattccagc atcctcacca tgacctgtat cagcgtggag 360
   cgcttcctgg gggtcctgta cccgctcagc tccaagcgct ggcgccgccg tcgttacgcg 420
   gtggccgcgt gtgcagggac ctggctgctg ctcctgaccg ccctgtgccc gctggcgcgc 480
   accgatctca cctacccggt gcacgccctg ggcatcatca cctgcttcga cgtcctcaag 540
   tggacgatgc tccccagcgt ggccatgtgg gccgtgttcc tcttcaccat cttcatcctg 600
   ctgttcctca tcccgttcgt gatcaccgtg gcttgttaca cggccaccat cctcaagctg 660
   ttgcgcacgg aggaggcgca cggccgggag cagcggaggc gcgcggtggg cctggccgcg 720
   gtggtcttgc tggcctttgt cacctgcttc gcccccaaca acttcgtgct cctggcgcac 780
   atcgtgagcc gcctgttcta cggcaagagc tactaccacg tgtacaagct cacgctgtgt 840
   ctcagctgcc tcaacaactg tctggacccg tttgtttatt actttgcgtc ccgggaattc 900
   cagctgcgcc tgcgggaata tttgggctgc cgccgggtgc ccagagacac cctggacacg 960
   cgccgcgaga gcctcttctc cgccaggacc acgtccgtgc gctccgaggc cggtgcgcac 1020
   cctgaaggga tggagggagc caccaggccc ggcctccaga ggcaggagag tgtgttctga 1080
<210> 28
   <211> 359
   <212> PRT
   <213> Homo sapiens
<400> 28
   Met Gln Val Pro Asn Ser Thr Gly Pro Asp Asn Ala Thr Leu Gln Met
    1               5                  10                  15
   Leu Arg Asn Pro Ala Ile Ala Val Ala Leu Pro Val Val Tyr Ser Leu
               20                  25                  30
   Val Ala Ala Val Ser Ile Pro Gly Asn Leu Phe Ser Leu Trp Val Leu
           35                  40                  45
   Cys Arg Arg Met Gly Pro Arg Ser Pro Ser Val Ile Phe Met Ile Asn
       50                  55                  60
   Leu Ser Val Thr Asp Leu Met Leu Ala Ser Val Leu Pro Phe Gln Ile
    65                  70                  75                  80
   Tyr Tyr His Cys Asn Arg His His Trp Val Phe Gly Val Leu Leu Cys
                    85                  90                  95
   Asn Val Val Thr Val Ala Phe Tyr Ala Asn Met Tyr Ser Ser Ile Leu
               100                 105                 110
   Thr Met Thr Cys Ile Ser Val Glu Arg Phe Leu Gly Val Leu Tyr Pro
           115                 120                 125
   Leu Ser Ser Lys Arg Trp Arg Arg Arg Arg Tyr Ala Val Ala Ala Cys
       130                 135                 140
   Ala Gly Thr Trp Leu Leu Leu Leu Thr Ala Leu Cys Pro Leu Ala Arg
   145                 150                 155                 160
   Thr Asp Leu Thr Tyr Pro Val His Ala Leu Gly Ile Ile Thr Cys Phe
                   165                 170                 175
   Asp Val Leu Lys Trp Thr Met Leu Pro Ser Val Ala Met Trp Ala Val
               180                 185                 190
   Phe Leu Phe Thr Ile Phe Ile Leu Leu Phe Leu Ile Pro Phe Val Ile
           195                 200                 205
   Thr Val Ala Cys Tyr Thr Ala Thr Ile Leu Lys Leu Leu Arg Thr Glu
       210                 215                 220
   Glu Ala His Gly Arg Glu Gln Arg Arg Arg Ala Val Gly Leu Ala Ala
   225                 230                 235                 240
   Val Val Leu Leu Ala Phe Val Thr Cys Phe Ala Pro Asn Asn Phe Val
                   245                 250                 255
   Leu Leu Ala His Ile Val Ser Arg Leu Phe Tyr Gly Lys Ser Tyr Tyr
               260                 265                 270
   His Val Tyr Lys Leu Thr Leu Cys Leu Ser Cys Leu Asn Asn Cys Leu
           275                 280                 285
   Asp Pro Phe Val Tyr Tyr Phe Ala Ser Arg Glu Phe Gln Leu Arg Leu
       290                 295                 300
   Arg Glu Tyr Leu Gly Cys Arg Arg Val Pro Arg Asp Thr Leu Asp Thr
   305                 310                 315                 320
   Arg Arg Glu Ser Leu Phe Ser Ala Arg Thr Thr Ser Val Arg Ser Glu
                   325                 330                 335
   Ala Gly Ala His Pro Glu Gly Met Glu Gly Ala Thr Arg Pro Gly Leu
               340                 345                 350
   Gln Arg Gln Glu Ser Val Phe
           355
<210> 29
   <211> 1503
   <212> DNA
   <213> Homo sapiens
<400> 29
   atggagcgtc cctgggagga cagcccaggc ccggaggggg cagctgaggg ctcgcctgtg 60
   ccagtcgccg ccggggcgcg ctccggtgcc gcggcgagtg gcacaggctg gcagccatgg 120
   gctgagtgcc cgggacccaa ggggaggggg caactgctgg cgaccgccgg ccctttgcgt 180
   cgctggcccg ccccctcgcc tgccagctcc agccccgccc ccggagcggc gtccgctcac 240
   tcggttcaag gcagcgcgac tgcgggtggc gcacgaccag ggcgcagacc ttggggcgcg 300
   cggcccatgg agtcggggct gctgcggccg gcgccggtga gcgaggtcat cgtcctgcat 360
   tacaactaca ccggcaagct ccgcggtgcg agctaccagc cgggtgccgg cctgcgcgcc 420
   gacgccgtgg tgtgcctggc ggtgtgcgcc ttcatcgtgc tagagaatct agccgtgttg 480
   ttggtgctcg gacgccaccc gcgcttccac gctcccatgt tcctgctcct gggcagcctc 540
   acgttgtcgg atctgctggc aggcgccgcc tacgccgcca acatcctact gtcggggccg 600
   ctcacgctga aactgtcccc cgcgctctgg ttcgcacggg agggaggcgt cttcgtggca 660
   ctcactgcgt ccgtgctgag cctcctggcc atcgcgctgg agcgcagcct caccatggcg 720
   cgcagggggc ccgcgcccgt ctccagtcgg gggcgcacgc tggcgatggc agccgcggcc 780
   tggggcgtgt cgctgctcct cgggctcctg ccagcgctgg gctggaattg cctgggtcgc 840
   ctggacgctt gctccactgt cttgccgctc tacgccaagg cctacgtgct cttctgcgtg 900
   ctcgccttcg tgggcatcct ggccgcgatc tgtgcactct acgcgcgcat ctactgccag 960
   gtacgcgcca acgcgcggcg cctgccggca cggcccggga ctgcggggac cacctcgacc 1020
   cgggcgcgtc gcaagccgcg ctctctggcc ttgctgcgca cgctcagcgt ggtgctcctg 1080
   gcctttgtgg catgttgggg ccccctcttc ctgctgctgt tgctcgacgt ggcgtgcccg 1140
   gcgcgcacct gtcctgtact cctgcaggcc gatcccttcc tgggactggc catggccaac 1200
   tcacttctga accccatcat ctacacgctc accaaccgcg acctgcgcca cgcgctcctg 1260
   cgcctggtct gctgcggacg ccactcctgc ggcagagacc cgagtggctc ccagcagtcg 1320
   gcgagcgcgg ctgaggcttc cgggggcctg cgccgctgcc tgcccccggg ccttgatggg 1380
   agcttcagcg gctcggagcg ctcatcgccc cagcgcgacg ggctggacac cagcggctcc 1440
   acaggcagcc ccggtgcacc cacagccgcc cggactctgg tatcagaacc ggctgcagac 1500
   tga                                                                1503
<210> 30
   <211> 500
   <212> PRT
   <213> Homo sapiens
<400> 30
   Met Glu Arg Pro Trp Glu Asp Ser Pro Gly Pro Glu Gly Ala Ala Glu
   1               5                   10                  15
   Gly Ser Pro Val Pro Val Ala Ala Gly Ala Arg Ser Gly Ala Ala Ala
               20                  25                  30
   Ser Gly Thr Gly Trp Gln Pro Trp Ala Glu Cys Pro Gly Pro Lys Gly
           35                  40                  45
   Arg Gly Gln Leu Leu Ala Thr Ala Gly Pro Leu Arg Arg Trp Pro Ala
       50                  55                  60
   Pro Ser Pro Ala Ser Ser Ser Pro Ala Pro Gly Ala Ala Ser Ala His
   65                  70                  75                  80
   Ser Val Gln Gly Ser Ala Thr Ala Gly Gly Ala Arg Pro Gly Arg Arg
                   85                  90                  95
   Pro Trp Gly Ala Arg Pro Met Glu Ser Gly Leu Leu Arg Pro Ala Pro
               100                 105                 110
   Val Ser Glu Val Ile Val Leu His Tyr Asn Tyr Thr Gly Lys Leu Arg
           115                 120                 125
   Gly Ala Ser Tyr Gln Pro Gly Ala Gly Leu Arg Ala Asp Ala Val Val
       130                 135                 140
   Cys Leu Ala Val Cys Ala Phe Ile Val Leu Glu Asn Leu Ala Val Leu
   145                 150                 155                 160
   Leu Val Leu Gly Arg His Pro Arg Phe His Ala Pro Met Phe Leu Leu
                   165                 170                 175
   Leu Gly Ser Leu Thr Leu Ser Asp Leu Leu Ala Gly Ala Ala Tyr Ala
               180                 185                 190
   Ala Asn Ile Leu Leu Ser Gly Pro Leu Thr Leu Lys Leu Ser Pro Ala
           195                 200                 205
   Leu Trp Phe Ala Arg Glu Gly Gly Val Phe Val Ala Leu Thr Ala Ser
       210                 215                 220
   Val Leu Ser Leu Leu Ala Ile Ala Leu Glu Arg Ser Leu Thr Met Ala
   225                 230                 235                 240
   Arg Arg Gly Pro Ala Pro Val Ser Ser Arg Gly Arg Thr Leu Ala Met
                   245                 250                 255
   Ala Ala Ala Ala Trp Gly Val Ser Leu Leu Leu Gly Leu Leu Pro Ala
               260                 265                 270
   Leu Gly Trp Asn Cys Leu Gly Arg Leu Asp Ala Cys Ser Thr Val Leu
           275                 280                 285
   Pro Leu Tyr Ala Lys Ala Tyr Val Leu Phe Cys Val Leu Ala Phe Val
       290                 295                 300
   Gly Ile Leu Ala Ala Ile Cys Ala Leu Tyr Ala Arg Ile Tyr Cys Gln
   305                 310                 315                 320
   Val Arg Ala Asn Ala Arg Arg Leu Pro Ala Arg Pro Gly Thr Ala Gly
                   325                 330                 335
   Thr Thr Ser Thr Arg Ala Arg Arg Lys Pro Arg Ser Leu Ala Leu Leu
               340                 345                 350
   Arg Thr Leu Ser Val Val Leu Leu Ala Phe Val Ala Cys Trp Gly Pro
           355                 360                 365
   Leu Phe Leu Leu Leu Leu Leu Asp Val Ala Cys Pro Ala Arg Thr Cys
       370                 375                 380
   Pro Val Leu Leu Gln Ala Asp Pro Phe Leu Gly Leu Ala Met Ala Asn
   385                 390                 395                 400
   Ser Leu Leu Asn Pro Ile Ile Tyr Thr Leu Thr Asn Arg Asp Leu Arg
                   405                 410                 415
   His Ala Leu Leu Arg Leu Val Cys Cys Gly Arg His Ser Cys Gly Arg
                   420                     425                     430
   Asp Pro Ser Gly Ser Gln Gln Ser Ala Ser Ala Ala Glu Ala Ser Gly
           435                     440                     445
   Gly Leu Arg Arg Cys Leu Pro Pro Gly Leu Asp Gly Ser Phe Ser Gly
       450                     455                     460
   Ser Glu Arg Ser Ser Pro Gln Arg Asp Gly Leu Asp Thr Ser Gly Ser
   465                     470                     475                     480
   Thr Gly Ser Pro Gly Ala Pro Thr Ala Ala Arg Thr Leu Val Ser Glu
                   485                     490                     495
   Pro Ala Ala Asp
               500
<210> 31
   <211> 1029
   <212> DNA
   <213> Homo sapiens
<400> 31
   atgcaagccg tcgacaatct cacctctgcg cctgggaaca ccagtctgtg caccagagac 60
   tacaaaatca cccaggtcct cttcccactg ctctacactg tcctgttttt tgttggactt 120
   atcacaaatg gcctggcgat gaggattttc tttcaaatcc ggagtaaatc aaactttatt 180
   atttttctta agaacacagt catttctgat cttctcatga ttctgacttt tccattcaaa 240
   attcttagtg atgccaaact gggaacagga ccactgagaa cttttgtgtg tcaagttacc 300
   tccgtcatat tttatttcac aatgtatatc agtatttcat tcctgggact gataactatc 360
   gatcgctacc agaagaccac caggccattt aaaacatcca accccaaaaa tctcttgggg 420
   gctaagattc tctctgttgt catctgggca ttcatgttct tactctcttt gcctaacatg 480
   attctgacca acaggcagcc gagagacaag aatgtgaaga aatgctcttt ccttaaatca 540
   gagttcggtc tagtctggca tgaaatagta aattacatct gtcaagtcat tttctggatt 600
   aatttcttaa ttgttattgt atgttataca ctcattacaa aagaactgta ccggtcatac 660
   gtaagaacga ggggtgtagg taaagtcccc aggaaaaagg tgaacgtcaa agttttcatt 720
   atcattgctg tattctttat ttgttttgtt cctttccatt ttgcccgaat tccttacacc 780
   ctgagccaaa cccgggatgt ctttgactgc actgctgaaa atactctgtt ctatgtgaaa 840
   gagagcactc tgtggttaac ttccttaaat gcatgcctgg atccgttcat ctattttttc 900
   ctttgcaagt ccttcagaaa ttccttgata agtatgctga agtgccccaa ttctgcaaca 960
   tctctgtccc aggacaatag gaaaaaagaa caggatggtg gtgacccaaa tgaagagact 1020
   ccaatgtaa                                                         1029
<210> 32
   <211> 342
   <212> PRT
   <213> Homo sapiens
<400> 32
<210> 33
   <211> 1077
   <212> DNA
   <213> Homo sapiens
<400> 33
   atgtcggtct gctaccgtcc cccagggaac gagacactgc tgagctggaa gacttcgcgg 60
   gccacaggca cagccttcct gctgctggcg gcgctgctgg ggctgcctgg caacggcttc 120
   gtggtgtgga gcttggcggg ctggcggcct gcacgggggc gaccgctggc ggccacgctt 180
   gtgctgcacc tggcgctggc cgacggcgcg gtgctgctgc tcacgccgct ctttgtggcc 240
   ttcctgaccc ggcaggcctg gccgctgggc caggcgggct gcaaggcggt gtactacgtg 300
   tgcgcgctca gcatgtacgc cagcgtgctg ctcaccggcc tgctcagcct gcagcgctgc 360
   ctcgcagtca cccgcccctt cctggcgcct cggctgcgca gcccggccct ggcccgccgc 420
   ctgctgctgg cggtctggct ggccgccctg ttgctcgccg tcccggccgc cgtctaccgc 480
   cacctgtgga gggaccgcgt atgccagctg tgccacccgt cgccggtcca cgccgccgcc 540
   cacctgagcc tggagactct gaccgctttc gtgcttcctt tcgggctgat gctcggctgc 600
   tacagcgtga cgctggcacg gctgcggggc gcccgctggg gctccgggcg gcacggggcg 660
   cgggtgggcc ggctggtgag cgccatcgtg cttgccttcg gcttgctctg ggccccctac 720
   cacgcagtca accttctgca ggcggtcgca gcgctggctc caccggaagg ggccttggcg 780
   aagctgggcg gagccggcca ggcggcgcga gcgggaacta cggccttggc cttcttcagt 840
   tctagcgtca acccggtgct ctacgtcttc accgctggag atctgctgcc ccgggcaggt 900
   ccccgtttcc tcacgcggct cttcgaaggc tctggggagg cccgaggggg cggccgctct 960
   agggaaggga ccatggagct ccgaactacc cctcagctga aagtggtggg gcagggccgc 1020
   ggcaatggag acccgggggg tgggatggag aaggacggtc cggaatggga cctttga 1077
<210> 34
   <211> 358
   <212> PRT
   <213> Homo sapiens
<400> 34
   Met Ser Val Cys Tyr Arg Pro Pro Gly Asn Glu Thr Leu Leu Ser Trp
   1               5                   10                  15
   Lys Thr Ser Arg Ala Thr Gly Thr Ala Phe Leu Leu Leu Ala Ala Leu
               20                  25                  30
   Leu Gly Leu Pro Gly Asn Gly Phe Val Val Trp Ser Leu Ala Gly Trp
           35                  40                  45
   Arg Pro Ala Arg Gly Arg Pro Leu Ala Ala Thr Leu Val Leu His Leu
       50                  55                  60
   Ala Leu Ala Asp Gly Ala Val Leu Leu Leu Thr Pro Leu Phe Val Ala
   65                  70                  75                  80
   Phe Leu Thr Arg Gln Ala Trp Pro Leu Gly Gln Ala Gly Cys Lys Ala
                   85                  90                  95
   Val Tyr Tyr Val Cys Ala Leu Ser Met Tyr Ala Ser Val Leu Leu Thr
               100                 105                 110
   Gly Leu Leu Ser Leu Gln Arg Cys Leu Ala Val Thr Arg Pro Phe Leu
           115                 120                 125
   Ala Pro Arg Leu Arg Ser Pro Ala Leu Ala Arg Arg Leu Leu Leu Ala
       130                 135                 140
   Val Trp Leu Ala Ala Leu Leu Leu Ala Val Pro Ala Ala Val Tyr Arg
   145                 150                 155                 160
   His Leu Trp Arg Asp Arg Val Cys Gln Leu Cys His Pro Ser Pro Val
                   165                 170                 175
   His Ala Ala Ala His Leu Ser Leu Glu Thr Leu Thr Ala Phe Val Leu
               180                 185                 190
   Pro Phe Gly Leu Met Leu Gly Cys Tyr Ser Val Thr Leu Ala Arg Leu
           195                 200                 205
   Arg Gly Ala Arg Trp Gly Ser Gly Arg His Gly Ala Arg Val Gly Arg
       210                 215                 220
   Leu Val Ser Ala Ile Val Leu Ala Phe Gly Leu Leu Trp Ala Pro Tyr
   225                 230                 235                 240
   His Ala Val Asn Leu Leu Gln Ala Val Ala Ala Leu Ala Pro Pro Glu
                   245                 250                 255
   Gly Ala Leu Ala Lys Leu Gly Gly Ala Gly Gln Ala Ala Arg Ala Gly
               260                 265                 270
   Thr Thr Ala Leu Ala Phe Phe Ser Ser Ser Val Asn Pro Val Leu Tyr
           275                 280                 285
   Val Phe Thr Ala Gly Asp Leu Leu Pro Arg Ala Gly Pro Arg Phe Leu
       290                 295                 300
   Thr Arg Leu Phe Glu Gly Ser Gly Glu Ala Arg Gly Gly Gly Arg Ser
   305                 310                 315                 320
   Arg Glu Gly Thr Met Glu Leu Arg Thr Thr Pro Gln Leu Lys Val Val
                   325                 330                 335
   Gly Gln Gly Arg Gly Asn Gly Asp Pro Gly Gly Gly Met Glu Lys Asp
               340                 345                 350
   Gly Pro Glu Trp Asp Leu
           355
<210> 35
   <211> 1005
   <212> DNA
   <213> Homo sapiens
<400> 35
   atgctgggga tcatggcatg gaatgcaact tgcaaaaact ggctggcagc agaggctgcc 60
   ctggaaaagt actacctttc cattttttat gggattgagt tcgttgtggg agtccttgga 120
   aataccattg ttgtttacgg ctacatcttc tctctgaaga actggaacag cagtaatatt 180
   tatctcttta acctctctgt ctctgactta gcttttctgt gcaccctccc catgctgata 240
   aggagttatg ccaatggaaa ctggatatat ggagacgtgc tctgcataag caaccgatat 300
   gtgcttcatg ccaacctcta taccagcatt ctctttctca cttttatcag catagatcga 360
   tacttgataa ttaagtatcc tttccgagaa caccttctgc aaaagaaaga gtttgctatt 420
   ttaatctcct tggccatttg ggttttagta accttagagt tactacccat acttcccctt 480
   ataaatcctg ttataactga caatggcacc acctgtaatg attttgcaag ttctggagac 540
   cccaactaca acctcattta cagcatgtgt ctaacactgt tggggttcct tattcctctt 600
   tttgtgatgt gtttctttta ttacaagatt gctctcttcc taaagcagag gaataggcag 660
   gttgctactg ctctgcccct tgaaaagcct ctcaacttgg tcatcatggc agtggtaatc 720
   ttctctgtgc tttttacacc ctatcacgtc atgcggaatg tgaggatcgc ttcacgcctg 780
   gggagttgga agcagtatca gtgcactcag gtcgtcatca actcctttta cattgtgaca 840
   cggcctttgg cctttctgaa cagtgtcatc aaccctgtct tctattttct tttgggagat 900
   cacttcaggg acatgctgat gaatcaactg agacacaact tcaaatccct tacatccttt 960
   agcagatggg ctcatgaact cctactttca ttcagagaaa agtga                 1005
<210> 36
   <211> 334
   <212> PRT
   <213> Homo sapiens
<400> 36
   Met Leu Gly Ile Met Ala Trp Asn Ala Thr Cys Lys Asn Trp Leu Ala
   1               5                   10                  15
   Ala Glu Ala Ala Leu Glu Lys Tyr Tyr Leu Ser Ile Phe Tyr Gly Ile
               20                  25                  30
   Glu Phe Val Val Gly Val Leu Gly Asn Thr Ile Val Val Tyr Gly Tyr
           35                  40                  45
   Ile Phe Ser Leu Lys Asn Trp Asn Ser Ser Asn Ile Tyr Leu Phe Asn
       50                  55                  60
   Leu Ser Val Ser Asp Leu Ala Phe Leu Cys Thr Leu Pro Met Leu Ile
   65                  70                  75                  80
   Arg Ser Tyr Ala Asn Gly Asn Trp Ile Tyr Gly Asp Val Leu Cys Ile
                   85                  90                  95
   Ser Asn Arg Tyr Val Leu His Ala Asn Leu Tyr Thr Ser Ile Leu Phe
               100                 105                 110
   Leu Thr Phe Ile Ser Ile Asp Arg Tyr Leu Ile Ile Lys Tyr Pro Phe
           115                 120                 125
   Arg Glu His Leu Leu Gln Lys Lys Glu Phe Ala Ile Leu Ile Ser Leu
       130                 135                 140
   Ala Ile Trp Val Leu Val Thr Leu Glu Leu Leu Pro Ile Leu Pro Leu
   145                 150                 155                 160
   Ile Asn Pro Val Ile Thr Asp Asn Gly Thr Thr Cys Asn Asp Phe Ala
                   165                 170                 175
   Ser Ser Gly Asp Pro Asn Tyr Asn Leu Ile Tyr Ser Met Cys Leu Thr
               180                 185                 190
   Leu Leu Gly Phe Leu Ile Pro Leu Phe Val Met Cys Phe Phe Tyr Tyr
           195                 200                 205
   Lys Ile Ala Leu Phe Leu Lys Gln Arg Asn Arg Gln Val Ala Thr Ala
       210                 215                 220
   Leu Pro Leu Glu Lys Pro Leu Asn Leu Val Ile Met Ala Val Val Ile
   225                 230                 235                 240
   Phe Ser Val Leu Phe Thr Pro Tyr His Val Met Arg Asn Val Arg Ile
   245 250 255
   Ala Ser Arg Leu Gly Ser Trp Lys Gln Tyr Gln Cys Thr Gln Val Val
   260 265 270
   Ile Asn Ser Phe Tyr Ile Val Thr Arg Pro Leu Ala Phe Leu Asn Ser
   275 280 285
   Val Ile Asn Pro Val Phe Tyr Phe Leu Leu Gly Asp His Phe Arg Asp
   290 295 300
   Met Leu Met Asn Gln Leu Arg His Asn Phe Lys Ser Leu Thr Ser Phe
   305 310 315 320
   Ser Arg Trp Ala His Glu Leu Leu Leu Ser Phe Arg Glu Lys
   325 330
<210> 37
   <211> 1296
   <212> DNA
   <213> Homo sapiens
<400> 37
   atgcaggcgc ttaacattac cccggagcag ttctctcggc tgctgcggga ccacaacctg 60
   acgcgggagc agttcatcgc tctgtaccgg ctgcgaccgc tcgtctacac cccagagctg 120
   ccgggacgcg ccaagctggc cctcgtgctc accggcgtgc tcatcttcgc cctggcgctc 180
   tttggcaatg ctctggtgtt ctacgtggtg acccgcagca aggccatgcg caccgtcacc 240
   aacatcttta tctgctcctt ggcgctcagt gacctgctca tcaccttctt ctgcattccc 300
   gtcaccatgc tccagaacat ttccgacaac tggctggggg gtgctttcat ttgcaagatg 360
   gtgccatttg tccagtctac cgctgttgtg acagaaatgc tcactatgac ctgcattgct 420
   gtggaaaggc accagggact tgtgcatcct tttaaaatga agtggcaata caccaaccga 480
   agggctttca caatgctagg tgtggtctgg ctggtggcag tcatcgtagg atcacccatg 540
   tggcacgtgc aacaacttga gatcaaatat gacttcctat atgaaaagga acacatctgc 600
   tgcttagaag agtggaccag ccctgtgcac cagaagatct acaccacctt catccttgtc 660
   atcctcttcc tcctgcctct tatggtgatg cttattctgt acagtaaaat tggttatgaa 720
   ctttggataa agaaaagagt tggggatggt tcagtgcttc gaactattca tggaaaagaa 780
   atgtccaaaa tagccaggaa gaagaaacga gctgtcatta tgatggtgac agtggtggct 840
   ctctttgctg tgtgctgggc accattccat gttgtccata tgatgattga atacagtaat 900
   tttgaaaagg aatatgatga tgtcacaatc aagatgattt ttgctatcgt gcaaattatt 960
   ggattttcca actccatctg taatcccatt gtctatgcat ttatgaatga aaacttcaaa 1020
   aaaaatgttt tgtctgcagt ttgttattgc atagtaaata aaaccttctc tccagcacaa 1080
   aggcatggaa attcaggaat tacaatgatg cggaagaaag caaagttttc cctcagagag 1140
   aatccagtgg aggaaaccaa aggagaagca ttcagtgatg gcaacattga agtcaaattg 1200
   tgtgaacaga cagaggagaa gaaaaagctc aaacgacatc ttgctctctt taggtctgaa 1260
   ctggctgaga attctccttt agacagtggg cattaa 1296
<210> 38
   <211> 431
   <212> PRT
   <213> Homo sapiens
<400> 38
   Met Gln Ala Leu Asn Ile Thr Pro Glu Gln Phe Ser Arg Leu Leu Arg
    1               5                  10                  15
   Asp His Asn Leu Thr Arg Glu Gln Phe Ile Ala Leu Tyr Arg Leu Arg
   20 25 30
   Pro Leu Val Tyr Thr Pro Glu Leu Pro Gly Arg Ala Lys Leu Ala Leu
   35 40 45
   Val Leu Thr Gly Val Leu Ile Phe Ala Leu Ala Leu Phe Gly Asn Ala
   50 55 60
   Leu Val Phe Tyr Val Val Thr Arg Ser Lys Ala Met Arg Thr Val Thr
   65 70 75 80
   Asn Ile Phe Ile Cys Ser Leu Ala Leu Ser Asp Leu Leu Ile Thr Phe
   85 90 95
   Phe Cys Ile Pro Val Thr Met Leu Gln Asn Ile Ser Asp Asn Trp Leu
   100 105 110
   Gly Gly Ala Phe Ile Cys Lys Met Val Pro Phe Val Gln Ser Thr Ala
   115 120 125
   Val Val Thr Glu Met Leu Thr Met Thr Cys Ile Ala Val Glu Arg His
   130 135 140
   Gln Gly Leu Val His Pro Phe Lys Met Lys Trp Gln Tyr Thr Asn Arg
   145 150 155 160
   Arg Ala Phe Thr Met Leu Gly Val Val Trp Leu Val Ala Val Ile Val
   165 170 175
   Gly Ser Pro Met Trp His Val Gln Gln Leu Glu Ile Lys Tyr Asp Phe
   180 185 190
   Leu Tyr Glu Lys Glu His Ile Cys Cys Leu Glu Glu Trp Thr Ser Pro
   195 200 205
   Val His Gln Lys Ile Tyr Thr Thr Phe Ile Leu Val Ile Leu Phe Leu
   210 215 220
   Leu Pro Leu Met Val Met Leu Ile Leu Tyr Ser Lys Ile Gly Tyr Glu
   225 230 235 240
   Leu Trp Ile Lys Lys Arg Val Gly Asp Gly Ser Val Leu Arg Thr Ile
   245 250 255
   His Gly Lys Glu Met Ser Lys Ile Ala Arg Lys Lys Lys Arg Ala Val
   260 265 270
   Ile Met Met Val Thr Val Val Ala Leu Phe Ala Val Cys Trp Ala Pro
   275 280 285
   Phe His Val Val His Met Met Ile Glu Tyr Ser Asn Phe Glu Lys Glu
   290 295 300
   Tyr Asp Asp Val Thr Ile Lys Met Ile Phe Ala Ile Val Gln Ile Ile
   305 310 315 320
   Gly Phe Ser Asn Ser Ile Cys Asn Pro Ile Val Tyr Ala Phe Met Asn
   325 330 335
   Glu Asn Phe Lys Lys Asn Val Leu Ser Ala Val Cys Tyr Cys Ile Val
               340                 345                 350
   Asn Lys Thr Phe Ser Pro Ala Gln Arg His Gly Asn Ser Gly Ile Thr
           355                 360                 365
   Met Met Arg Lys Lys Ala Lys Phe Ser Leu Arg Glu Asn Pro Val Glu
       370                 375                 380
   Glu Thr Lys Gly Glu Ala Phe Ser Asp Gly Asn Ile Glu Val Lys Leu
   385                 390                 395                 400
   Cys Glu Gln Thr Glu Glu Lys Lys Lys Leu Lys Arg His Leu Ala Leu
                   405                 410                 415
   Phe Arg Ser Glu Leu Ala Glu Asn Ser Pro Leu Asp Ser Gly His
               420                 425                 430
<210> 39
   <211> 24
   <212> DNA
   <213> Homo sapiens
<400> 39
   ctgtgtacag cagttcgcag agtg 24
<210> 40
   <211> 24
   <212> DNA
   <213> Homo sapiens
<400> 40
   gagtgccagg cagagcaggt agac 24
<210> 41
   <211> 31
   <212> DNA
   <213> Homo sapiens
<400> 41
   cccgaattcc tgcttgctcc cagcttggcc c 31
<210> 42
   <211> 32
   <212> DNA
   <213> Homo sapiens
<400> 42
   tgtggatcct gctgtcaaag gtcccattcc gg 32
<210> 43
   <211> 20
   <212> DNA
   <213> Homo sapiens
<400> 43
   tcacaatgct aggtgtggtc 20
<210> 44
   <211> 22
   <212> DNA
   <213> Homo sapiens
<400> 44
   tgcatagaca atgggattac ag 22
<210> 45
   <211> 511
   <212> DNA
   <213> Homo sapiens
<400> 45
   tcacaatgct aggtgtggtc tggctggtgg cagtcatcgt aggatcaccc atgtggcacg 60
   tgcaacaact tgagatcaaa tatgacttcc tatatgaaaa ggaacacatc tgctgcttag 120
   aagagtggac cagccctgtg caccagaaga tctacaccac cttcatcctt gtcatcctct 180
   tcctcctgcc tcttatggtg atgcttattc tgtacgtaaa attggttatg aactttggat 240
   aaagaaaaga gttggggatg gttcagtgct tcgaactatt catggaaaag aaatgtccaa 300
   aatagccagg aagaagaaac gagctgtcat tatgatggtg acagtggtgg ctctctttgc 360
   tgtgtgctgg gcaccattcc atgttgtcca tatgatgatt gaatacagta attttgaaaa 420
   ggaatatgat gatgtcacaa tcaagatgat ttttgctatc gtgcaaatta ttggattttc 480
   caactccatc tgtaatccca ttgtctatgc a 511
<210> 46
   <211> 21
   <212> DNA
   <213> Homo sapiens
<400> 46
   ctgcttagaa gagtggacca g 21
<210> 47
   <211> 22
   <212> DNA
   <213> Homo sapiens
<400> 47
   ctgtgcacca gaagatctac ac 22
<210> 48
   <211> 21
   <212> DNA
   <213> Homo sapiens
<400> 48
   caaggatgaa ggtggtgtag a 21
<210> 49
   <211> 23
   <212> DNA
   <213> Homo sapiens
<400> 49 23
   gtgtagatct tctggtgcac agg 23
<210> 50
   <211> 21
   <212> DNA
   <213> Homo sapiens
<400> 50 21
   gcaatgcagg tcatagtgag c 21
<210> 51
   <211> 27
   <212> DNA
   <213> Homo sapiens
<400> 51
   tggagcatgg tgacgggaat gcagaag 27
<210> 52
   <211> 27
   <212> DNA
   <213> Homo sapiens
<400> 52
   gtgatgagca ggtcactgag cgccaag 27
<210> 53
   <211> 23
   <212> DNA
   <213> Homo sapiens
<400> 53
   gcaatgcagg cgcttaacat tac 23
<210> 54
   <211> 22
   <212> DNA
   <213> Homo sapiens
<400> 54
   ttgggttaca atctgaaggg ca 22
<210> 55
   <211> 23
   <212> DNA
   <213> Homo sapiens
<400> 55
   actccgtgtc cagcaggact ctg 23
<210> 56
   <211> 24
   <212> DNA
   <213> Homo sapiens
<400> 56
   tgcgtgttcc tggaccctca cgtg 24
<210> 57
   <211> 29
   <212> DNA
   <213> Homo sapiens
<400> 57
   caggccttgg attttaatgt cagggatgg 29
<210> 58
   <211> 27
   <212> DNA
   <213> Homo sapiens
<400> 58
   ggagagtcag ctctgaaaga attcagg 27
<210> 59
   <211> 27
   <212> DNA
   <213> Homo sapiens
<400> 59
   tgatgtgatg ccagatacta atagcac 27
<210> 60
   <211> 27
   <212> DNA
   <213> Homo sapiens
<400> 60
   cctgattcat ttaggtgaga ttgagac 27
<210> 61
   <211> 21
   <212> DNA
   <213> Homo sapiens
<400> 61
   gacaggtacc ttgccatcaa g 21
<210> 62
   <211> 22
   <212> DNA
   <213> Homo sapiens
<400> 62
   ctgcacaatg ccagtgataa gg 22
<210> 63
   <211> 27
   <212> DNA
   <213> Homo sapiens
<400> 63
   ctgacttctt gttcctggca gcagcgg 27
<210> 64
   <211> 27
   <212> DNA
   <213> Homo sapiens
<400> 64
   agaccagcca gggcacgctg aagagtg 27
<210> 65
   <211> 32
   <212> DNA
   <213> Homo sapiens
<400> 65
   gatcaagctt ccatcctact gaaaccatgg tc 32
<210> 66
   <211> 35
   <212> DNA
   <213> Homo sapiens
<400> 66
   gatcagatct cagttccaat attcacacca ccgtc 35
<210> 67
   <211> 22
   <212> DNA
   <213> Homo sapiens
<400> 67
   ctggtgtgct ccatggcatc cc 22
<210> 68
   <211> 22
   <212> DNA
   <213> Homo sapiens
<400> 68
   gtaagcctcc cagaacgaga gg 22
<210> 69
   <211> 24
   <212> DNA
   <213> Homo sapiens
<400> 69
   cagcgcaggg tgaagcctga gagc 24
<210> 70
   <211> 24
   <212> DNA
   <213> Homo sapiens
<400> 70
   ggcacctgct gtgacctgtg cagg 24
<210> 71
   <211> 22
   <212> DNA
   <213> Homo sapiens
<400> 71
   gtcctgccac ttcgagacat gg 22
<210> 72
   <211> 23
   <212> DNA
   <213> Homo sapiens
<400> 72
   gaaacttctc tgcccttacc gtc 23
<210> 73
   <211> 26
   <212> DNA
   <213> Homo sapiens
<400> 73
   ccaacaccag catccatggc atcaag 26
<210> 74
   <211> 27
   <212> DNA
   <213> Homo sapiens
<400> 74
   ggagagtcag ctctgaaaga attcagg 27